(19) Europäisches Patentamt
European Patent Office
Office européen des brevets

(11) Publication number: **0 140 684 B1**

(12) **EUROPEAN PATENT SPECIFICATION**

(45) Date of publication of patent specification: **11.07.90**

(21) Application number: **84307390.9**

(22) Date of filing: **26.10.84**

(51) Int. Cl.[5]: **C 07 C 69/738,** C 07 C 59/90, C 07 D 307/93, C 07 C 321/28, C 07 C 381/10, C 07 C 317/00, A 61 K 31/34, A 61 K 31/35, A 61 K 31/215

(54) **Leukotriene antagonists.**

(30) Priority: **27.10.83 US 546013**
**23.01.84 US 573169**

(43) Date of publication of application:
**08.05.85 Bulletin 85/19**

(45) Publication of the grant of the patent:
**11.07.90 Bulletin 90/28**

(84) Designated Contracting States:
**CH DE FR GB IT LI NL**

(56) References cited:
**EP-A-0 061 800**
**EP-A-0 083 228**
**EP-A-0 106 565**
**GB-A-2 058 785**

(73) Proprietor: **MERCK FROSST CANADA INC.**
**16711 Trans-Canada Highway**
**Kirkland Quebec (CA)**

(72) Inventor: **Frenette, Richard**
**1915 de Linbourg Vimont**
**Laval Quebec (CA)**
Inventor: **Rokach, Joshua**
**416 Place Canterbury Chomedey**
**Laval Quebec (CA)**
Inventor: **Young, Robert N.**
**216 Senneville Road**
**Senneville Quebec (CA)**
Inventor: **Kakushima, Masatoshi**
**10-4 Hirochi-machi**
**Isogo-ku Yokohama (JP)**

(74) Representative: **Crampton, Keith John Allen et al**
**D YOUNG & CO 10 Staple Inn**
**London WC1V 7RD (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 140 684 B1

Courier Press, Leamington Spa, England.

## Description

This invention is directed to compounds which act as antagonists of the leukotrienes.

The leukotrienes are a novel group of biologically active mediators derived from arachidonic acid through the action of lipoxygenase enzyme systems. There are two groups of leukotrienes derived from the common unstable precursor Leukotriene $A_4$. The first of these are the peptido-lipid leukotrienes, the most important being Leukotrienes $C_4$ and $D_4$. These compounds collectively account for the biologically active material known as the slow reacting substance of anaphylaxis.

The leukotrienes are potent smooth muscle contracting agents, particularly on respiratory smooth muscle but also on other tissues (e.g. gall bladder). In addition, they promote mucous production, modulate vascular permeability changes and are potent inflammatory agents in human skin. The most important compound in the second group of leukotrienes is Leukotriene $B_4$, a dihydroxy fatty acid. This compound is a potent chemotactic agent for neutrophils and eosinophils and in addition, may modulate a number of other functions of these cells. It also effects other cell types such as lymphocytes and for example may modulate the action of T-suppressor cells and natural killer cells. When injected *in vivo,* in addition to promoting the accumulation of leukocytes, Leukotriene $B_4$ is also a potent hyperalgesic agent and can modulate vascular permeability changes through a neutrophil dependent mechanism. Both groups of leukotrienes are formed following oxygenation of arachidonic acid through the action of a 5-lipoxygenase enzyme. See for example, D. M. Bailey *et al., Ann. Rpts. Med. Chem. 17* 203 (1982).

The leukotrienes are potent spasmogens of human trachea, bronchus and lung parenchymal strips, and when administered to normal volunteers as aerosols are 3,800 times more potent than histamine at inducing a 50% decrease in air flow at 30% of vital capacity. They mediate increases in vascular permeability to animals and promote mucous production in human bronchial explants. In addition, Leukotriene $B_4$ may also mediate mucous production and could be an important mediator of neutrophil and eosinophil accumulation in asthmatic lungs. 5-Lipoxygenase products are also thought to be regulators of mast cell degranulation and recent studies with human lung mast cells have suggested that 5-lipoxygenase inhibitors, but not corticosteroids, may suppress antigen-induced mast cell degranulation. *In vitro* studies have shown that antigen challenge of human lung results in the release of leukotrienes and in addition purified human mast cells can produce substantial amount of leukotrienes. There is therefore good evidence that leukotrienes are important mediators of human asthma. Leukotriene antagonists would therefore be a new class of drugs for the treatment of asthma.

Psoriasis is a human skin disease which effects between two and six percent of the population. There is no adequate therapy for psoriasis and related skin conditions. The evidence for leukotriene involvement in these diseases is as follows. One of the earliest events in the development of prepapillary lesions is the recruitment of leukocytes to the skin site. Injection of Leukotriene $B_4$ into human skin results in a pronounced neutrophil accumulation. There are gross abnormalities in arachidonic acid metabolism in human psoriatic skin. In particular, highly elevated levels of free arachidonic acid can be measured as well as large amounts of lipoxygenase products. Leukotriene $B_4$ has been detected in psoriatic lesions, but not in uninvolved skin, in biologically significant amounts.

Leukotrienes can be measured in nasal washings from patients with allergic rhinitis and are greatly elevated following antigen challenge. Leukotrienes may mediate this disease through their ability to regulate mast cell degranulation, by modulating mucous production and mucocillary clearance and be mediating the accumulation of inflammatory leukocytes.

Leukotrienes can also mediate other diseases. These include atopic dermatitis, gouty arthritis and gall bladder spasms. In addition, they may have a role in cardiovascular disease because leukotrienes $C_4$ and $D_4$ act as coronary and cerebral arterial vasconstrictors and these compounds may also have negative inotropic effects on the myocardium. In addition, the leukotrienes are important mediators of inflammatory diseases through their ability to modulate leukocyte and lymphocyte function. See for example, B. Samuelson, *Science, 220,* 568 (1983).

Several classes of compounds exhibit ability to antagonize the action of leukotrienes in mammals, especially humans. See for example: United Kingdom Patent Specification GB—A—2 058 785; and European Patent Applications EP—A—0 056 172, 0 061 800 and 0 083 228.

EP—A—0 083 228 discloses pharmaceutically active phenylcarboxylic acid derivatives that antagonize the slow-reacting substance of anaphylaxis (SRS-A) compounds thereof, e.g., leukotrienes, in warm-blooded animals. The compounds are diphenyl carboxylic acids with particular linking groups between the

individual phenyl rings and the carboxylic acid residue, and are particularly useful in relieving asthma and inflammation in man. The compounds have the formula

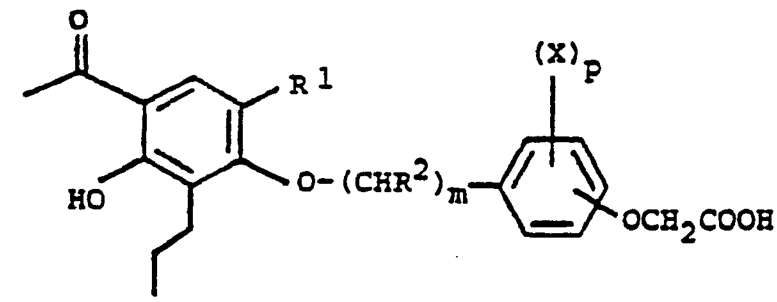

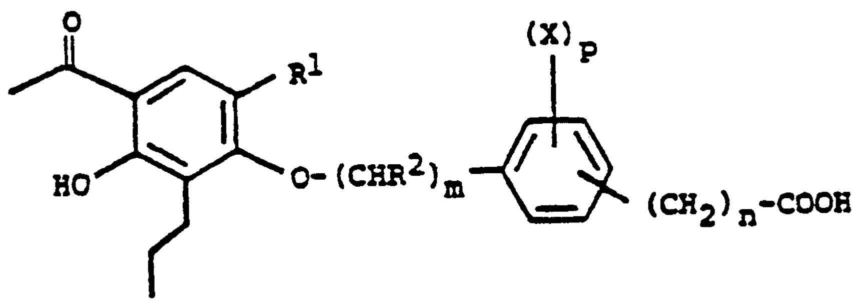

or

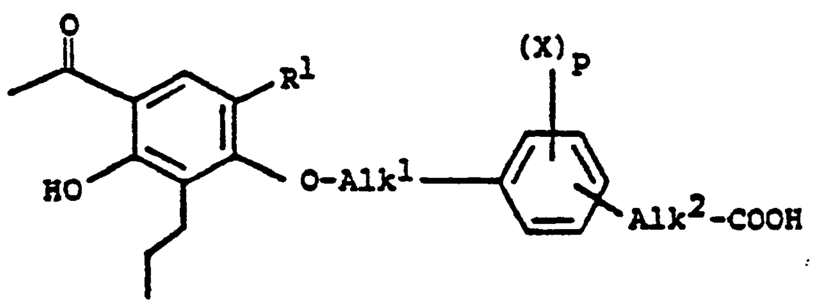

in which $R^1$ is hydrogen or iodine; $R^2$ is hydrogen, alkyl, phenyl or substituted phenyl;

each X is halogen, nitro, hydroxy, alkyl or alkoxy;

each $Alk^1$ and $Alk^2$ is a direct bond, alkenyl, substituted alkenyl, alkylene or substituted alkylene, at least one of them being alkenyl or substituted alkenyl;

each of m and n is 0, 1, 2 or 3, and

p is 0, 1 or 2

or are their pharmaceutically acceptable base-addition salts.

EP—A—0 061 800 discloses other anti-SRS-A carboxylic acid derivatives of formula

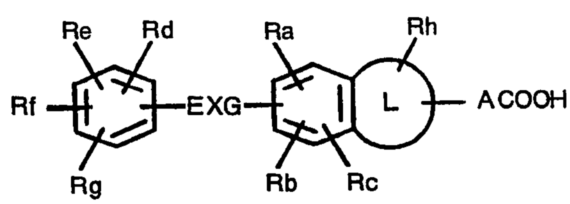

in which each of Ra, Rb, Rc, Rd, Re, Rf and Rg, which may be same or different, represents hydrogen, amino, hydroxy, alkoxy, alkenloxy, halogen, acyl, alkenyl, alkyl, or alkoxy substituted by phenyl,

Rh is hydrogen, alkyl or —COOH,

X is hydrocarbon chain containing from 2 to 10 carbon atoms and optionally substituted by a hydroxy group,

A has no significance or represents Y, OY, or SY, and Y represents a $C_{1-4}$ hydrocarbon chain which is optionally substituted by $C_{1-4}$ alkyl,

each of E and G, which may be the same or different, represents —O—, —S— or —$CH_2$—, provided that at least one of E and G is —O— or —S—,

L, together with the carbon atoms to which it is attached, forms a benzene, furan, thiophene, indole, or pyran-2-one ring,

and pharmaceutically acceptable derivatives thereof.

The present invention provides compounds that act as antagonists to prevent leukotriene action or as inhibitors to prevent synthesis. These compounds may be administered by insufflation, intravenously, rectally, topically, orally, parenterally including subcutaneously and intramuscularly, or nasally. The present invention also provides methods for the preparation of these compounds. The present invention also provides intermediates useful in the synthesis of these compounds. The present invention also provides pharmaceutical formulations for administering these compounds.

The compounds of the present invention may be used to treat or prevent mammalian (especially human) disease states such as erosive gastritis; erosive esophagitis; inflammatory bowel disease; ethanol-induced hemorrhagic erosions; hepatic ischenmia; noxious agent induced damage or necrosis of hepatic, pancreatic, renal, or myocardial tissue; liver parenchymal damage caused by hepatoxic agents such as $CCl_4$ and D-galactosamine; ischemic renal failure, disease-induced hepatic damage; bile salt induced pancreatic or gastric damage; trauma- or stress-induced cell damage; and glycerol-induced renal failure.

The present invention concerns compounds having activity as leukotriene antagonists, methods for their preparation, and methods for using these compounds. Because of their activity as leukotriene antagonists, the compounds of the present invention are useful as anti-asthmatic, anti-allergic, and anti-inflammatory agents and are useful in treating allergic rhinitis and chronic bronchitis and for amelioration of skin diseases like psoriasis and atopic exzema. These compounds are useful to antagonize or inhibit the pathologic actions of leukotrienes on the cardiovascular and vascular systems.

The compounds of the present invention have the formula:

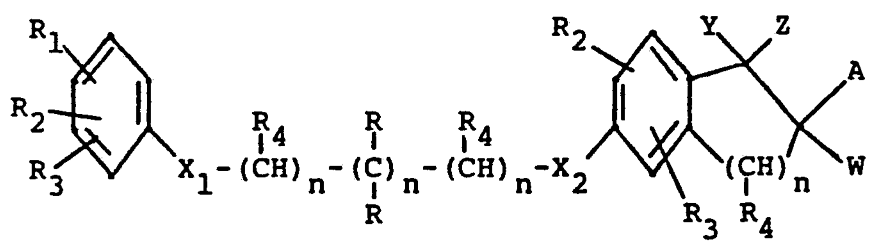

I

in which each R, independently of the other(s), is H, OH or $OR_2$, or $C(R)_2$ represents $C{=}C(R_4)_2$;

$R_1$ is H, OH, $R_4CO$, or $R_5OCO$;

each of $R_2$ and $R_3$, independently of the others, is H, OH, $C_{1-6}$ alkyl, $C_{2-6}$ alkenyl, trifluoromethyl, $C_{1-6}$ alkoxy, SH, $C_{1-6}$ thioalkyl, phenyl, phenyl having $C_{1-3}$ alkyl or halogen substitution, benzyl, phenethyl, halogen, amino, $N(R_4)_2$, $COOR_4$, $CH_2OR_4$, formyl, CN, trifluoromethylthio or nitro;

each of $X_1$ and $X_2$, independently of the other, is oxygen, sulfur, sulfoxide, sulfone, $—SO{=}NR_5$, $NR_6$, $N—CO—R_7$, N—CN or $NCONHR_6$;

each of $R_4$ and $R_6$, independently of the other(s), is H or $C_{1-6}$ alkyl;

each $R_5$ is $C_{1-6}$ alkyl;

each $R_7$, independently of the other, if any, is $C_{1-6}$ alkyl or $C_{1-6}$ alkoxy;

W is H or $C_{1-6}$ alkyl or is combined with Y to form an unsaturated bond;

Z is H, OH is $C_{1-6}$ alkoxy, or is combined with Y to form an oxo residue;

Y, if not combined with Z or W, is hydrogen;

A is $—(C(R_4)_2)_s—R_8$ where s is 0, 1, 2 or 3 and $R_8$ is $COOR_4$, $CH_2OH$, CHO, tetrazolyl, $NHSO_2R_9$, $CONHSO_2R_9$, hydroxymethylketone, CN, $CON(R_7)_2$, a monocyclic or bicyclic heterocyclic ring containing an acidic hydroxyl group, or

$$-COO(CH_2)_s-\underset{R_6}{\overset{R_6}{C}}-(CH_2)_s-R_{10}$$

where each s, independently of the others, is 0, 1, 2 or 3 and $R_{10}$ is (A) a monocyclic or bicyclic heterocyclic radical containing from 3 to 12 nuclear carbon atoms and, as nuclear heteroatoms, two Ns or one N and one S, and with each ring in the heterocyclic radical being formed of 5 or 6 atoms, or (B) the radical $X'—R_{11}$ where X' is O, S or NH and $R_{11}$ contains up to 21 carbon atoms and is a hydrocarbon radical or an acyl radical of an organic acyclic or monocyclic carboxylic acid containing not more than 1 heteroatom in the ring;

each n, independently of the others, is 0, 1, 2 or 3;

$R^9$ is OH, $C_{1-6}$ alkyl, $C_{1-6}$ alkoxy, $C_{1-6}$ perfluoroalkyl, phenyl, or phenyl having one or more $R^2$ substituents; or are their salts or acid-addition salts. In addition, when Z is OH and A is a carboxylic acid (or a derivative thereof) the compound may form a lactone structure, as illustrated in Scheme III. These lactone derivatives are also included within the scope of the present invention.

In preferred compounds,

$R_1$ is H, OH, or $R_4CO$;

$X_1$ and $X_2$ are each independently oxygen, sulfur, sulfoxide or sulfone; and

A is $-(C(R_4)_2)_s-R_8$ where s is 0, 1, 2 or 3 and $R_8$ is $COOR_4$, $CH_2OH$, CHO, tetrazolyl, $NHSO_2R_9$, $CONHSO_2R_9$, hydroxymethylketone, CN, or $CON(R_7)_2$;

and the other variables are as defined above.

Particularly preferred compounds of the present invention have the formula Ib:

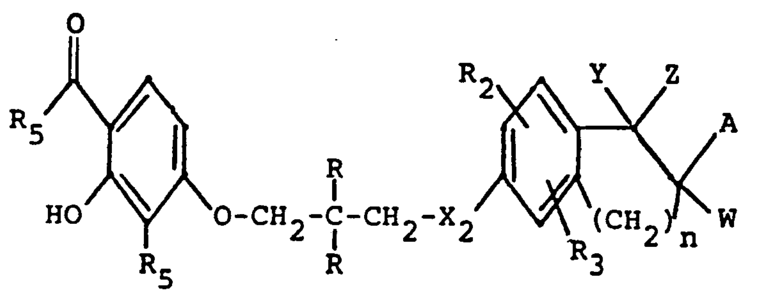

Ib

in which A is $-(C(R_4)_2)_s-R_8$ where s is 1, 2 or 3 and $R_8$ is $COOR_4$; $CH_2OH$; CHO; or tetrazolyl; n is 1, 2, or 3, and the other variables are as defined above.

All of the above definitions contemplate that, unless otherwise indicated, each $R_4$ and each $R_7$ in groups such as $=(R_4)_2$, $N(R_4)_2$ or $CON(R_7)_2$ can be the same as or different from one another.

SCHEME I

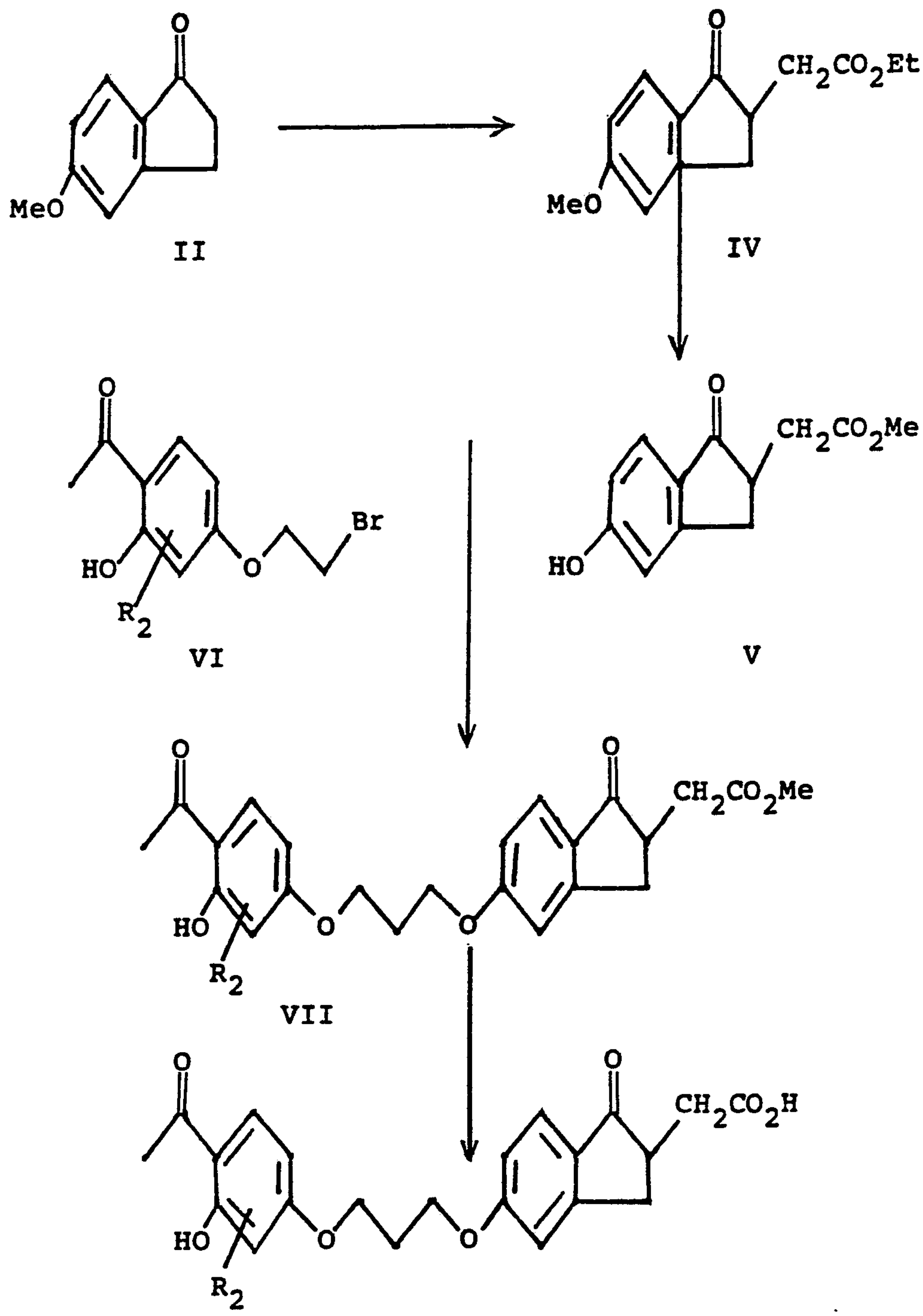

II

IV

VI

V

VII

I

The compounds of the present invention may be prepared by several different routes. According to one method, illustrated in Scheme I, a compound of formula II is reacted with ethyl bromoacetate in the presence of a base such as LDA, KH, or $KN(SiMe_3)_3$ to yield ethyl 5-methoxy-1-indanone-2-acetate, formula IV. Treatment of compound IV with (a) HBr/AcOH and (b) HCl/MeOH affords the 5-hydroxy-indanone compound of formula V.

Reaction of the compound V with the bromide of compound VI or its corresponding chloride or iodide, in the presence of a base, such as potassium carbonate, in a solvent such as methyl ethyl ketone, affords the compound VII. Other suitable bases are alkali metal carbonates such as $Li_2CO_3$, or $Na_2CO_3$. The reaction

may also be carried out in other solvents such as THF, monoglyme, diglyme, acetone, or DMF. The temperature range to carry out this transformation is 25—160°C, the optimum being 60—70°C.

The ester compound of formula VII is readily hydrolyzed to form the desired carboxylic acid of formula I by treatment with dilute aqueous base, for example, 1N NaOH followed by acidification with aqueous mineral acid, for example, aqueous HCl.

In addition to 5-methoxy-1-indanone, other similar compounds may be employed as starting materials in Scheme I reactions. For example, 5-methoxy-1-indanone-3-acetic acid may be substituted for compound IV in Scheme I esterified using 10% HCl/MeOH, forming the 3-methyl acetate isomer of the compound of formula V. In addition, 5,6- or 7-methoxy-1-tetralone compounds are commercially available (Aldrich) and may be readily substituted for the compound of formula II in Scheme I.

SCHEME II

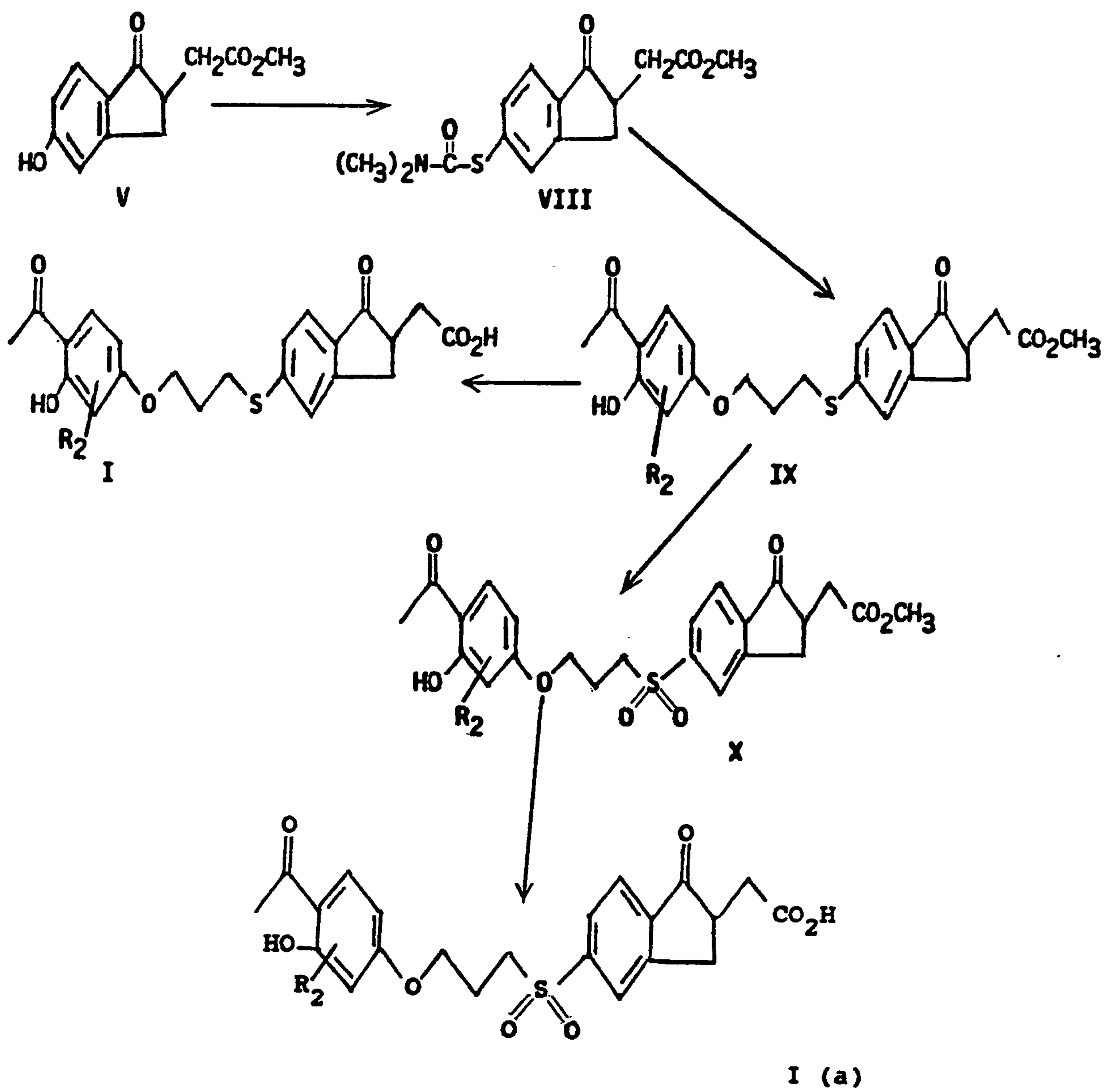

V

VIII

I

IX

X

I (a)

An alternate preparation of I as illustrated in Scheme II involves the reaction of the thiol from formula VIII with a compound of formula VI under the conditions already used successfully for reacting compounds V and VI. The compound VIII is readily available by reaction of compound V with dimethylthiocarbamoyl chloride in the presence of a base such as pyridine, $K_2CO_3$, or NaH, followed by heating the unisolated intermediate to 200°C. The thiol compound is available from VIII by hydrolysis. The intermediate ester of

structure IX is then hydrolyzed to I by reacting it with a base followed by treatment with acid. The corresponding sulfone of formula I(a) is available through the oxidation of compound IX with m-chloroperbenzoic acid (m-CPBA) and by hydrolysis of the sulfone of formula X.

In addition to the thiol compound of formula VIII, the corresponding tetralone compounds previously described in connection with Scheme I may be used to prepare the corresponding tetralones of the instant invention.

SCHEME III

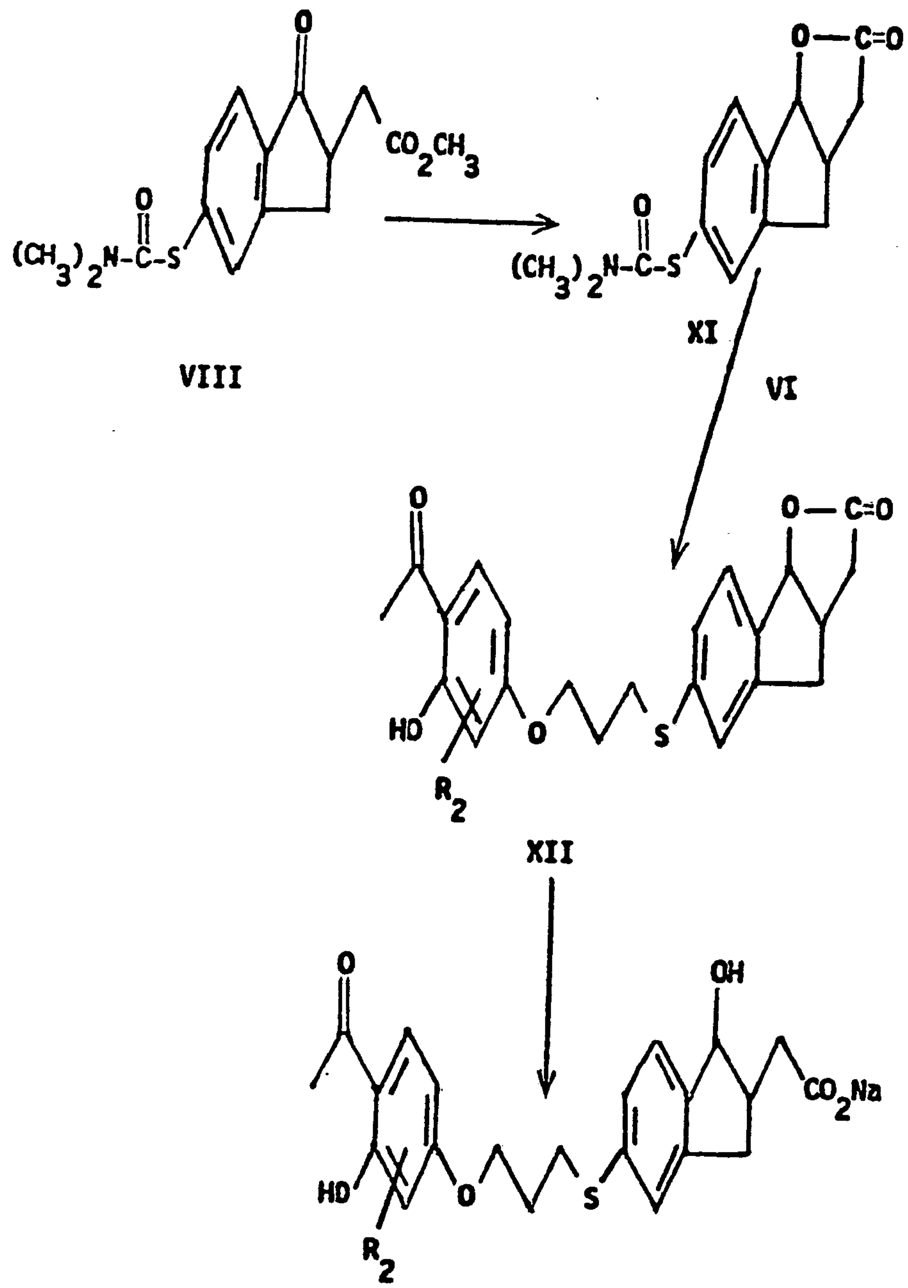

VIII

XI

VI

XII

I(b)

For the preparation of compounds of formula I where Z is OH, Scheme III is followed. Thus, reduction of the sodium salt prepared from the compound of formula VIII and 1N NaOH, with a reducing agent such as $NaBH_4$, gives rise, on acid work-up, to the gamma lactone of formula XI. Treatment of the gamma-lactone of formula XI as in Scheme III gives rise to the compound of formula XII. Base hydrolysis of the compound of formula XII, for example with 1N NaOH, affords the compound of formula I(b) wherein Z is OH.

In those instances when asymmetric carbon atoms are present, more than one stereoisomer is

possible, and all possible isomeric forms are deemed to be included within the planar structural representations shown. Optically active (R) and (S) isomers may be resolved using conventional techniques known to the skilled artisan.

The cytoprotective activity of a compound may be observed in both animal and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that cytoprotective compounds will prevent gastric lesions induced by oral administration of strong acids, strong bases, ethanol, hypertonic saline solutions and the like.

Two assays can be used to measure cytoprotective ability. These assays are: (A) an ethanol-induced lesion assay and (B) an indomethacin-induced ulcer assay.

A. Ethanol-Induced Gastric Ulcer Assay

Twenty-four hour fasted Sprague-Dawley (S.D.) rats are perorally (p.o.) dosed with 1.0 ml absolute ethanol. Fifteen to thirty minutes prior to ethanol administration, groups of rats each receive either an aqueous vehicle (aqueous methylcellulose 5% wt.) or the test compound at various doses perorally. One hour later, the animals are sacrificed and stomach mucosa are examined for resulting lesions.

B. Indomethacin-Induced Ulcer Assay

Indomethacin, 10 mg/kg p.o., is used to induce ulcers in 24 hour fasted S. K. rats. Fifteen minutes prior to indomethacin administration, groups of rats each receive either an aqueous vehicle (5% by weight methylcellulose) or the test compound at various doses perorally. Four hours later the animals are sacrificed and stomach mucosa are examined for resulting ulcers.

The magnitude of a prophylactic or therapeutic dose of a compound of formula I will, of course, vary with the nature of the severity of the condition to be treated and with the particular compound of formula I and its route of administration. In general, the daily dose range for anti-asthmatic, anti-allergic or anti-inflammatory use and, generally, uses other than cytoprotection lies within the range of from about 0.2 mg to about 100 mg to about 100 mg per kg body weight of a mammal. This dosage may be administered in single or divided individual doses.

As cytoprotective agents, the leukotriene antagonists of Formula I may generally be administered at a dosage range of 0.02 mg/kg to 100 mg/kg of body weight. The exact amount of inhibitor to be used will depend on, *inter alia,* whether it is being administered to heal damaged cells or to avoid future damage, on the nature of the damaged cells (e.g., gastro-intestinal ulcerations vs. nephrotic necrosis), and on the nature of the causative agent.

An example for the use of a compound of the Formula I in avoiding future damage would be coadministration of a compound of the Formula I with a non-steroidal anti-inflammatory drug (NSAID) that might otherwise cause such damage (for example, indomethacin). For such use, the compound of formula I is administered from 30 minutes prior up to 30 minutes after administration of NSAID. Preferably, it is administered prior to or simultaneously with the NSAID (for example in a combination dosage form).

The effective daily dosage level for compounds of Formulae I inducing cytoprotection in mammals, especially humans, will range from about 0.02 mg/kg to about 100 mg/kg, preferably from about 0.02 mg/kg to about 30 mg/kg. The dosage may be administered in single or divided individual doses.

Any suitable route of administration may be employed for providing a mammal, especially a human with an effective dosage of a leukotriene antagonist. For example, oral, rectal, transdermal, parenteral, intramuscular, intravenous and the like may be employed. Dosage forms include tablets, troches, dispersions, suspensions, solutions, capsules and the like.

The pharmaceutical compositions of the present invention comprise a compound of formula I as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. Salts derived from inorganic bases include sodium, potassium, lithium, ammonium, calcium, magnesium, ferrous, zinc, copper, manganous, aluminum, ferric, manganic salts and the like. Particularly preferred are the ammonium, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, tromethamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. For a helpful discussion of pharmaceutical salts see S. M. Berge *et al.,* Journal of Pharmaceutical Sciences, *66,* 1—19 (1977), the disclosure of which is hereby incorporated herein by reference.

The compositions of the present invention include compositions suitable for oral, rectal, ophthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently

presented in unit dosage form and prepared by any of the methods well-known in the art of pharmacy.

For use where a composition for intravenous administration is employed, a suitable dosage range for anti-asthmatic, anti-inflammatory or anti-allergic use is from about 0.2 mg to about 20 mg (preferably from about 1 to about 10 mg) of a compound of Formula I per kg of body weight per day and for cytoprotective use from about 0.02 mg to about 30 (preferably from about 0.02 to about 20) of a compound of Formula I per kg of body weight per day. In the case where an oral composition is employed, a suitable dosage range for anti-asthmatic, anti-inflammatory or anti-allergic use is from about 1 to about 100 mg of a compound of Formula I per kg of body weight per day, preferably from about 5 mg to about 40 mg per kg and for cytoprotective use from about 0.1 mg to about 30 mg (preferably from about 0.1 mg to about 20 mg) of a compound of Formula I per kg of body weight per day.

For administration by inhalation, the compounds of the present invention are conveniently delivered in the form of an aerosol spray presentation from pressurized packs or a nebuliser. The preferred composition for inhalation is a powder which may be formulated as a cartridge from which the powder composition may be inhaled with the aid of a suitable device. In the case of a pressurized aerosol, the dosage unit may be determined by providing a valve to deliver a metered amount.

In practical use, the compounds of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or intravenous. In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the compounds of Formula I may also be administered by controlled release means and/or delivery devices such as those described in U.S. Patent Nos. 3,845,770; 3,916,899; 3,536,809; 3,598,123; 3,630,200 and 4,008,719, the disclosure of which is hereby incorporated herein by reference.

Pharmaceutical compositions of the present invention suitable for oral administration and by inhalation in the case of asthma therapy may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Molded tablets may be made by molding in a suitable machine, a mixture of the powdered compound moistened with an inert liquid diluent. Desirably, each tablet contains from about 25 mg to about 500 mg of the active ingredient and each cachet or capsule contains from about 25 to about 500 mg of the active ingredient.

The following are examples of representative pharmaceutical dosage forms for the compounds of the present invention:

| Injectable Suspension | mg/ml |
|---|---|
| Leukotriene antagonist | 2.0 |
| Methylcellulose | 5.0 |
| Tween 80 0.5 | |
| Benzyl alcohol | 9.0 |
| Methyl paraben | 1.8 |
| Propyl paraben | 0.2 |
| Water for injection to a total volume of 1 ml | |

| Tablet | mg/tablet |
|---|---|
| Leukotriene antagonist | 25.0 |
| Microcrystalline Cellulose | 325.0 |
| Providone | 14.0 |
| Microcrystalline Cellulose | 90.0 |
| Pregelatinized Starch | 43.5 |
| Magnesium Stearate | 2.5 |
| | 500 |

| Capsule | mg/capsule |
|---|---|
| Leukotriene antagonist | 25.0 |
| Lactose Powder | 573.5 |
| Magnesium Stearate | 1.5 |
| | 600 |

In addition to the compounds of Formula I, the pharmaceutical compositions can also contain other active ingredients, such as cyclooxygenase inhibitors, non-steroidal anti-inflammatory drugs (NSAIDs), peripheral analgesic agents such as zomepirac diflunisal and the like. The weight ratio of the compound of the Formula I to the second active ingredient may be varied and will depend upon the effective dose of each ingredient. Generally, an effective dose of each will be used. Thus, for example, when a compound of the Formula I is combined with an NSAID, the weight ratio of the compound of the Formula I to the NSAID will generally range from about 200:1 to about 1:200. Combinations of a compound of the Formula I and other active ingredients will generally also be within the aforementioned range, but in each case, an effective dose of each active ingredient should be used.

NSAIDs can be characterized into five groups:

(1) the propionic acid derivatives;
(2) the acetic acid derivatives;
(3) the fenamic acid derivatives;
(4) the biphenylcarboxylic acid derivatives; and
(5) the oxicams

or a pharmaceutically acceptable salt thereof.

The propionic acid derivatives which may be used comprise: ibuprofen, ibuprufen aluminum, indoprofen, ketoprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, aminoprofen, tiaprofenic acid, fluprofen and bucloxic acid. Structurally related propionic acid derivatives having similar analgesic and anti-inflammatory properties are also intended to be included in this group.

Thus, "propionic acid derivatives" as defined herein are non-narcotic analgesics/non-steroidal anti-inflammatory drugs having a free —$CH(CH_3)COOH$ or —$CH_2CH_2COOH$ group (which optionally can be in the form of a pharmaceutically acceptable salt group, e.g., —$CH(CH_3)COO^-Na^+$ or —$CH_2CH_2COO^-Na^+$), typically attached directly or via a carbonyl function to a ring system, preferably to an aromatic ring system.

The acetic acid derivatives which may be used comprise: indomethacin, which is a preferred NSAID, sulindac, tolmetin, zomepirac, diclofenac, fenclofenac, alclofenac, ibufenac, isoxepac, furofenac, tiopinac, zidometacin, acemetacin, fentiazac, clidanac, oxpinac, and fenclozic acid. Structurally related acetic acid derivatives having similar analgesic and anti-inflammatory properties are also intended to be encompassed by this group.

Thus, "acetic acid derivatives" as defined herein are non-narcotic analgesics/non-steroidal anti-inflammatory drugs having a free —$CH_2COOH$ group (which optionally can be in the form of a pharmaceutically acceptable salt group, e.g. —$CH_2COO^-Na^+$), typically attached directly to a ring system, preferably to an aromatic or heteroaromatic ring system.

The fenamic acid derivatives which may be used comprise: mefenamic acid, meclofenamic acid, flufenamic acid, niflumic acid and tolfenamic acid. Structurally related fenamic acid derivatives having similar analgesic and anti-inflammatory properties are also intended to be encompassed by this group.

Thus, "fenamic acid derivatives" as defined herein are non-narcotic analgesics/non-steroidal anti-inflammatory drugs which contain the basic structure:

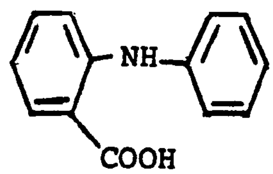

which can bear a variety of substituents and in which the free —COOH group can be in the form of a pharmaceutically acceptable salt group, e.g., —$COO^-Na^+$.

The biphenylcarboxylic acid derivatives which can be used comprise: diflunisal and flufenisal. Structurally related biphenylcarboxylic acid derivatives having similar analgesic and anti-inflammatory properties are also intended to be encompassed by this group.

Thus, "biphenylcarboxylic acid derivatives" as defined herein are non-narcotic analgesics/non-steroidal anti-inflammatory drugs which contain the basic structure:

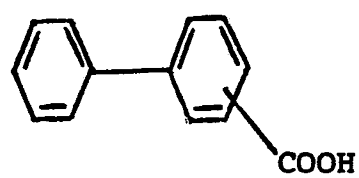

which can bear a variety of substituents and in which the free —COOH group can be in the form of a pharmaceutically acceptable salt group, e.g., —$COO^-Na^+$.

The oxicams which can be used in the present invention comprise: piroxicam, sudoxicam, isoxicam and 4-hydroxyl-1,2-benzothiazine 1,1-dioxide 4-(N-phenyl)-carboxamide. Structurally related oxicams having similar analgesic and anti-inflammatory properties are also intended to be encompassed by this group.

Thus, "oxicams" as defined herein are non-narcotic analgesics/non-steroidal anti-inflammatory drugs which have the general formula:

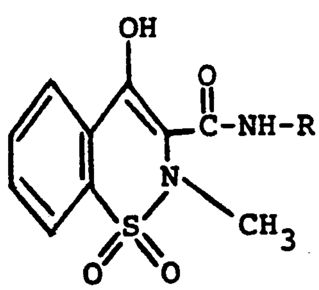

wherein R is an aryl or heteroaryl ring system.

The following NSAIDs may also be used: acemetacin, alminoprofen, amfenac sodium, aminoprofen, anitrazafen, antrafenine, auranofin, bendazac lysinate, benzydamine, beprozin, broperamole, bufezolac, carprofen, cinmetacin, ciproquazone, clidanac, cloximate, dazidamine, deboxamet, delmetacin, detomidine, dexindoprofen, diacerein, di-fisalamine, difenpyramide, emorfazone, enfenamic acid,

enolicam, epirizole, etersalate, etodolac, etofenamate, fanetizole mesylate, fenclofenac, fenclorac, fendosal, fenflumizole, fentiazac, feprazone, floctafenine, flunixin, flunoxaprofen, fluproquazone, fopirtoline, fosfosal, furcloprofen, furofenac, glucametacin, guaimesal, ibuproxam, isofezolac, isonixim, isoprofen, isoxepac, isoxicam, lefetamine HCl, leflunomide, lofemizole, lonazolac calcium, lotifazole, loxoprofen, lysin clonixinate, meclofenamate sodium, meseclazone, miroprofen, nabumetone, nictindole, nimesulide, orpanoxin, oxametacin, oxapadol, oxaprozin, perisoxal citrate, pimeprofen, pimetacin, piproxen, pirazolac, pirfenidone, pirprofen, pranoprofen, proglumetacin maleate, proquazone, pyridoxiprofen, sudoxicam, suprofen, talmetacin, talniflumate, tenoxicam, thiazolinobutazone, thielavin B, tiaprofenic acid, tiaramide HCl, tiflamizole, timegadine, tioxaprofen, tolfenamic acid, tolpadol, tryptamid, ufenamate, and zidometacin.

The following NSAIDs may also be used:

2-[4-(2-thiazolyloxy)-phenyl]-propionic acid
2,3,5-trimethyl-6-(12-hydroxy-5,10-dodecadiynyl-1,4-benzoquinone)
2-(8-methyl-10,11-dihydro-11-oxodibenz[b,f]oxepin-2-yl)propionic acid
3,4,5-trimethoxybenzoyl-ibuprofen
guaiphenesin ibuprofenate
[6-[[1-(3,4-dihydro-8-hydroxy-1-oxo-1H-2-benzopyran-3-yl)-3-methylbutyl]amino]-5-dihydroxy-6-oxo-3-hexanamido]ammonium chloride
chlorhexidine dinaproxenate
4′-acetamidophenyl-2-(5′-p-toluyl-1′-methyl-pyrrole)-acetate
(±)-5-benzoyl-2,3-dihydro-1H-pyrrolizine-1-carboxylic acid tromethamine salt
3-methylamino-1-[3-(trifluoromethyl)-phenyl)-2-pyrazoline
Chinoin-127, a rimazolium analogue
2-(p-methylallylaminophenyl)propionic acid
(±)-α-[[2-hydroxy-1,1-dimethylethyl)amino]methyl]benzyl alcohol)
5-[5-(4-chlorophenyl)-2-furanyl]-dihydro-2(3H)-furanone
mono(2-ammonium-2-hydroxymethyl-3,3-diol)(2R-cis)-(3-methyloxiranyl) phosphonate
diflunisal lysine salt
3,5-di-t-butyl-4-hydrobenzylidene-γ-butyrolactone
imidazole 2-hydroxybenzoate
methyl 5,6-E-5,6-methanoeicosa-7E,9E,11Z,14E-eicosatetranoate
2,4-diamino-7-methyl-pyrazolo(1,5-a)-1,3,5-triazine
2-(2′,4′-difluoro-4-biphenyl)oxypropionic acid
3-methyl-3-(4-acetyl-aminophenoxy)-2,4-dioxobenzocyclo-1-esanone
4-(p-chlorophenyl)-1-(p-fluorophenyl)pyrazole-3-acetic acid
2-aminomethyl-4-t-butyl-6-propionylphenol hydrochloride
glycolic acid [o-(2,6-dichloroanilino)phenyl]acetate ester
the 3-hydroxyphthalide ester of (±)-1-(p-chlorobenzoyl)-5-methoxy-2-methylindole-3-acetic acid
the phthalidyl ester of diclofenac
2,6-di-t-butyl-4-(2′-thenoyl)phenol
2-(4-(2-oxocyclohexylidenemethyl)phenyl]propionic acid
2-(4-biphenylyl)-4-hexenoic acid
11β,17,21-trihydroxy-6α-methylpregna-1,4-diene-3,20-dione 21-acetate 17-propionate
4-(2-bromo-4,5-dimethoxyphenyl)-octahydro-2H-quinolizin-2-one
N-(2-pyridyl)-2-methyl-4-cinnamoyloxy-2H-1,2-benzothiazine-3-carboxamide-1,1-dioxide
17α-ethylthio-9α-fluoro-11β-hydroxy-17β-methylthio-androsta-1,4-diene-3-one
1,1,3-trimethyl-5-phenylbiuret
1-(4-chlorbenzoyl-5-methoxy-2-methyl-1H-indole-3-acetic acid butanediol ester
2-(4-(3-methyl-2-butenyl)phenyl)propionic acid
3-ethyl-1-(3-nitrophenyl-2,4-[1H,3H]-quinazolindione
6,9-de-epoxy-6,9-(phenylimino)-delta(6,8)-PGI1
1-benzoyl-5-methoxy-2-methylindole-3-acetic acid
(5H-dibenzo[a,d]cyclohepten-5-ylidene)acetic acid

Finally, NSAIDs which may also be used include the salicylates, specifically aspirin, and the phenylbutazones, and pharmaceutically acceptable salts thereof.

Pharmaceutical compositions comprising the Formula I compounds may also contain inhibitors of the biosynthesis of the leukotrienes such as are disclosed in pending U.S. Patent Applications Serial Number 539,342, filed October 5, 1983, Serial Number 459,924, filed January 21, 1983, Serial Number 539,215, filed October 5, 1983, and Serial Number 547,161, filed October 31, 1983, which are hereby incorporated herein by reference.

The compounds of the Formula I may also be used in combination with leukotriene antagonists such as those disclosed in copending applications U.S. Serial Nos. 520,051 and 520,052, filed August 5, 1983 which are hereby incorporated herein by reference and others known in the art such as those disclosed in European Patent Application Nos. 56,172 and 61,800; and in U.K. Patent Specification No. 2,058,785, which are hereby incorporated herein by reference.

Pharmaceutical compositions comprising the Formula I compound may also contain as the second active ingredient, antihistaminic agents such as benadryl, dramamine, histadyl, phenergan and the like. Alternatively, they may include prostaglandin antagonists such as those disclosed in European Patent Application 11,067 or thromboxane antagonists such as those disclosed in U.S. 4,237,160. They may also contain histidine decarboxyase inhibitors such as α-fluoromethylhistidine, described in U.S. 4,325,961. The compounds of the Formula I may also be advantageously combined with an $H_1$ or $H_2$-receptor antagonist, such as for instance cimetidine, ranitidine, terfenadine, famotidine, aminothiadiazoles disclosed in EP 81102976.8 and like compounds, such as those disclosed in U.S. Patent Nos. 4,283,408; 4,362,736; 4,394,508; European Patent Application No. 40,696 and a pending application, U.S.S.N. 301,616, filed September 14, 1981. The pharmaceutical compositions may also contain a $K^+/H^+$ ATPase inhibitor such as omeprazole, disclosed in U.S. Pat. 4,255,431, and the like. Each of the references referred to in this paragraph is hereby incorporated herein by reference.

The following examples illustrate the present invention without, however, limiting the same thereto. All temperatures are expressed in degrees Celsius.

Example 1

Ethyl 5-methoxy-1-indanone-2-acetate

To a cooled (0°C) solution of distilled diisopropylamine (8.3 ml) in dry THF (50 ml) was added 1.6 M n-BuLi/hexane (34 ml). The reaction mixture was stirred at room temperature under $N_2$ for 15 minutes as a cloudy yellow solution. Then the mixture was cooled at −78°C (acetone/dry ice), and a solution of 5-methoxy-1-indanone (8 g) in dry THF (100 ml) was slowly added under $N_2$. A solid formed after complete addition. The mixture was stirred at −78°C under $N_2$ for 40 minutes. To this mixture was added dropwise ethyl bromoacetate (6.6 ml) and the mixture was slowly brought to room temperature and stirred overnight at room temperature under $N_2$ as a dark homogenous solution. The reaction mixture was poured into a mixture of $H_2O$ (500 ml), ice and conc. HCl. The mixture was extracted with $Et_2O$ (3X). The combined organic layers were washed with $H_2O$, dried and evaporated to give an oil. Chromatography of the oil on a column of silica gel (70—230 mesh, 200 g) using hexane/EtOAc as eluant gave the title compound.

Example 2

Methyl 5-hydroxy-1-indanone-2-acetate

A mixture of the compound of Example 1 (8.0 g), acetic acid (30 ml) and 48% HBr (150 ml) was refluxed overnight to afford a dark solution. The reaction mixture was cooled to room temperature and poured into a mixture of $H_2O$ and ice (500 ml) and extracted with EtOAc (3X). The combined organic layers were washed with $H_2O$ (3X), dried and evaporated to give a pink solid. The pink solid was suspended in methanol (20 ml), 10% HCl/MeOH (80 ml) was added and the reaction mixture was stirred overnight at room temperature. The reaction mixture was evaporated to yield the title compound as a pink solid, m.p. 146—150°.

Example 3

Methyl 5-(3-(4-acetyl-3-hydroxy-2-*n*-propylphenoxy)-propyloxy)-1-indanone-2-acetate

To a solution of 2-hydroxy-3-propyl-4-(3-bromopropoxy)acetophenone (1.1 g) and the compound of Example 2 (640 mg) in methyl ethyl ketone (30 ml) was added potassium carbonate (1.20 g). The resulting mixture was heated at reflux overnight. The reaction mixture was cooled to room temperature, filtered and the filtrate was evaporated. The residue was chromatographed on column of silica gel (70—230 mesh, 100 g) and eluted with toluene/EtOAc (10:2) to give the title compound. Purification by HPLC afforded a white solid, m.p. 64—66°.

Example 4

5-(3-(4-acetyl-3-hydroxy-2-*n*-propylphenoxy)propyloxy)-1-indanone-2-acetic acid

To a solution of the ester from Example 3 (830 mg) in THF (25 ml) was added 1N NaOH (5.5 ml) and MeOH (2 ml). The mixture was stirred at room temperature for 2 hours. The reaction mixture was diluted with $H_2O$ (200 ml), acidified with conc. HCl and extracted with EtOAc (3X). The combined organic extracts were washed with brine, dried and evaporated to give an oil. Purification of the oil on a column of silica gel (70—230 mesh, 50 g) eluting with toluene/dioxane/AcOH (10:2:0.1) gave the title compound as a beige solid, m.p. 132—134°.

| | | |
|---|---|---|
| Analysis, calculated: | C, 68.17; | H, 6.41. |
| Found: | C, 68.17; | H, 6.42. |

Example 5

Methyl 5-(N,N-dimethylcarbamylthio)-1-indanone-2-acetate

A solution of the compound of Example 2 (4.4 g) and dimethylthiocarbamoyl chloride (3.0 g) in pyridine (20 ml) was refluxed in an amtosphere of $N_2$ for 5 hours and cooled. The crude reaction mixture was poured into ice-water (500 ml), extracted with ether (200 ml × 3). The combined organic extracts were washed with 0.1N HCl, brine and concentrated. The residual oil in $CH_2Cl_2$ was washed with brine and dried over $Na_2SO_4$. After concentration *in vacuo,* the residual oil was heated (neat) at 200°C for 12 hours.

Purification of the resulting solid by HPLC, eluting with hexane/EtOAc (1:1), gave the title compound as an oil which crystallized when triturated with ether, m.p. 82—84°.

Example 6

Methyl 5-(3-(4-acetyl-3-hydroxy-2-*n*-propylphenoxy)propylthio)-1-indanone-2-acetate

To a solution of sodium (0.5 g) in MeOH (20 ml) at 0° was added the thiocarbamate of Example 5 (2.0 g) in one portion. The mixture was stirred at room temperature for 18 hours. 2-Hydroxy-3-propyl-4-(3-bromo-propoxy)acetophenone (2.1 g) was added and the mixture was stirred at room temperature for 8 hours. The reaction mixture was cooled to −20°C, poured into crushed ice, acidified with 1N HCl and extracted with $CH_2Cl_2$. The extracts were washed with brine and the acidic material was extracted into 0.1N NaOH, reacidified with 1N HCl and extracted with $CH_2Cl_2$ to yield Fraction A. The neutral material was washed with brine, dried over $Na_2SO_4$, to yield Fraction B, which was chromatographed on a column of silica gel (20—230 mesh, 100 g) eluting with (10:3) hexane-EtOAc then (2:1) hexane-EtOAc to give the title ester as a solid, m.p. 73—75°C.

Fraction A was esterified with MeOH—HCl (gas) at room temperature overnight and similarly chromatographed to give the title ester.

Example 7

5-(3-(4-acetyl-3-hydroxy-2-*n*-propylphenoxy)propylthio)-1-indanone-2-acetic acid

The ester of Example 6 (896 mg) was hydrolyzed at room temperature using 1N NaOH (5.7 ml) in THF (25 ml) for 2 hours and evaporated *in vacuo* at 30°. The residual aqueous layer was acidified with 1N HCl in an ice-bath. Extraction with EtOAc gave an oil which was crystallized from ether to give the title compound, m.p. 125—128°.

| | | | |
|---|---|---|---|
| Analysis, calculated: | C, 65.77; | H, 6.18; | S, 7.02. |
| Found: | C, 65.76; | H, 6.24; | S, 7.20. |

Example 8

Methyl 5-(3-(4-acetyl-3-hydroxy-2-*n*-propylphenoxy)propylsulfonyl)-1-indanone-2-acetate

To a solution of the ester of Example 6 (780 mg) in $CH_2Cl_2$ (10 ml) at 0° was added a solution of m-CPBA (900 mg) in $CH_2Cl_2$ (20 ml). The solution was stirred at room temperature for 20 minutes, $Ca(OH)_2$ (1 g) was added and the mixture was filtered. Removal of the solvent gave an oil and chromatography on a column of silica gel, eluting with hexane-EtOAc (1:1), gave the title compound as crystals, m.p. 97—100°.

Example 9

5-(3-(4-acetyl-3-hydroxy-2-*n*-propylphenoxy)propylsulfonyl)-1-indanone-2-acetic acid

The ester of Example 8 (692 mg) was hydrolyzed at room temperature for 2 hours using 1N NaOH (4.2 ml) in THF (20 ml). The reaction mixture was acified and extracted with EtOAc (50 ml × 3). The combined organic extracts were washed with brine, dried and concentrated *in vacuo* to give the title compound, m.p. 170—173°, after recrystallization from ether.

| | | | |
|---|---|---|---|
| Analysis, calculated: | C, 61.46; | H, 5.78; | S, 6.56. |
| Found: | C, 61.37; | H, 5.92; | S, 6.50 |

Example 10

5-(N,N-dimethylcarbamylthio)-1-hydroxyindane-2-acetic acid gamma lactone

To a solution of the thiocarbamate ketoester of Example 5 (615 mg) in THF (3 ml) was added 1N NaOH (2.5 ml) and MeOH (0.5 ml) and the mixture was stirred at room temperature for 1 hour. To the resulting sodium salt in solution were added successively 40 mg of $NaBH_4$ and 1 mg of $CeCl_3$ at 0°. ($H_2$ gas was evolved.) The mixture was stirred at 0° for 20 minutes and acidified with 1N HCl. Extraction with ether gave colorless solids. To the solid dissolved in $CH_2Cl_2$ was added trifluoroacetic acid (TFA) (2 drops) and the solution was stirred for 12 hours at room temperature. The solution was washed with aqueous bicarbonate, brine and dried over $Na_2SO_4$ to give the title compound as an oil.

NMR ($CDCl_3$) (ppm): 2.37 (1H, dd, J=18 and 5 Hz), 2.67—3.53 (10 H), 5.87 (1H, d, J=7 Hz), 7.47 (3H, m).

Example 11

5-(3-(4-acetyl-3-hydroxy-2-*n*-propylphenoxy)propylthio)-1-hydroxy-indane-2-acetic acid gamma lactone

To a solution of the compound of Example 10 (280 mg) in MeOH (10 ml) was added 2N NaOH (5 ml) and the solution was refluxed in an atmosphere of argon for 15 hours. The mixture was cooled and 2-hydroxy-3-propyl-4-(3-bromopropoxy)acetophenone (473 mg) was added at 20° and the mixture was stirred at 20° for 0.5 hours then refluxed for 4 hours. The mixture was cooled, poured into ice, acidified with 6N HCl and extracted with $CH_2Cl_2$. The combined extracts were concentrated, and the residue redissolved in 20 ml of $CH_2Cl_2$ and treated with 5 drops of TFA at 20°C for 15 minutes. The solution was washed with brine, dried over $Na_2SO_4$ and concentrated *in vacuo*. Chromatography in a column of silica gel (20—230 mesh) eluting with EtOAc-hexane (1:1) gave the title compound as an oil.

NMR ($CDCl_3$) (ppm): 0.93 (3H, t), 1.55 (2H, m), 2.20 (2H, m), 2.37—3.53 (12H, including 3H singlet at

2.55), 4.15 (2H, t), 5.83 (1H, d, J=7 Hz), 6.43 (1H, d, J=9 Hz), 7.30 (3H, m), 7.63 (1H, d, J=9 Hz), 12.83 (1H, exchangeable in $D_2O$).

Example 12

5-(3-(4-acetyl-3-hydroxy-2-*n*-propylphenoxy)propylthio)-1-hydroxy-indane-2-acetic acid sodium salt

A solution of the gamma-lactone from Example 11 (230 mg) in THF (5 ml), 1N NaOH (0.8 ml) and MeOH (1 ml) was stirred at 20° for 12 hours and concentrated to dryness. The residue, dissolved in $H_2O$, was passed through an XAD-8 column (eluting first with $H_2O$ then with 95% EtOH). The fractions containing the title salt were collected and concentrated to dryness.

| | | | |
|---|---|---|---|
| Analysis, calculated: | C, 62.48; | H, 6.08; | S, 6.67. |
| Found: | C, 62.52; | H, 6.05; | S, 6.67. |

Example 13

Methyl 6-methoxy-1-tetralone-2-acetate

To a solution of LDA, prepared from diisopropylamine (0.84 ml) and *n*-butyl lithium (3.44 ml, 1.6 M in hexane) in 10 ml of THF at −78°C was added dropwise a solution of 6-methoxy-1-tetralone (0.88 g, 5.0 mmol) in 5 ml of THF in an atmosphere of nitrogen. To the stirred solution at −78° was then added methyl bromoacetate (0.5 ml, 6.0 mmole) and the mixture was allowed to warm to room temperature. Aqueous work-up and extraction with ether gave, after chromatography on silica gel, the title compound, m.p. 110—112°.

Example 14

Methyl 6-hydroxy-1-tetralone-2-acetate

A solution of the 6-methoxy-1-tetralone derivative (860 mg) (from Example 13) in 47% HBr (15 ml) and glacial acetic acid (5 ml) was refluxed for 30 hours and poured into ice-water. The product was extracted into ethyl acetate (3X) and the combined organic extracts were washed with brine, dried over $Na_2SO_4$ and concentrated to dryness. The residue was esterified with 10% HCl in methanol to give, after work-up, the title compound, m.p. 121—123°.

Example 15

Methyl 6-(N,N-dimethylcarbamylthio)-1-tetralone-2-acetate

Following the procedure of Example 5, but substituting the compound of Example 14 for methyl 5-hydroxy-1-indanone-2-acetate, the title compound is obtained.

Example 16

Methyl 6-(3-(4-acetyl-3-hydroxy-2-*n*-propylphenoxy)propylthio)-1-tetralone-2-acetate

Following the procedure of Example 6, but substituting the compound of Example 15 for the compound of Example 5, the title compound is obtained.

Example 17

6-(3-(4-Acetyl-3-hydroxy-2-*n*-propylphenoxy)propylthio)-1-tetralone-2-acetic acid

Following the procedure of Example 7, but substituting the compound of Example 16 for the compound of Example 6, the title compound is obtained.

Example 18

Methyl 6-(3-(4-acetyl-3-hydroxy-2-*n*-propylphenoxy)propyloxy)-1-tetralone-2-acetate

Following the procedure of Example 3, but substituting the compound of Example 14 for methyl 5-hydroxy-1-indanone-2-acetate, the title compound was obtained as a solid, m.p. 96—98°.

| | | |
|---|---|---|
| Analysis, calculated: | C, 69.21; | H, 6.89. |
| Found: | C, 69.08; | H, 6.93. |

Example 19

6-(3-(4-acetyl-3-hydroxy-2-*n*-propylphenoxy)propyloxy)-1-tetralone-2-acetic acid

Following the procedure of Example 4, but substituting the compound of Example 18 for the compound of Example 3, the title compound was obtained as a solid, m.p. 123—125°.

| | | |
|---|---|---|
| Analysis, calculated: | C, 68.70; | H, 6.65. |
| Found: | C, 68.95; | H, 6.70. |

Example 20

Methyl 5-(3-bromopropyloxy)-1-indanone-2-acetate

A mixture of 1,3-dibromopropane (15 ml), the compound of of Example 2 (8.0 g) and potassium carbonate (15.0 g) in methyl ethyl ketone (45 ml) was heated at reflux overnight. The reaction mixture was filtered through a bed of Celite and the filtrate was concentrated in vacuo. The residue was chromatographed on a column of silica gel (70—230 mesh, 200 g) eluting with hexane/EtOAc (5:2) to give the title compound as an oil.

NMR ($CDCl_3$) (ppm): 2.2—3.5 (7H, m), 3.60 (2H, t, J=6 Hz), 3.67 (3H, s), 4.17 (2H, t, J=6 Hz), 6.80—7.00 (2H, m), 7.65 (1H, d, J=9 Hz).

Example 21

5-(3-Bromopropyloxy)-1-hydroxyindane-2-acetic acid gamma lactone

To a solution of the compound of Example 20 (8.7 g) and cerium (III) chloride (50 mg) in methanol (60 ml) at 0°C was added sodium borohydride (1.23 g). The reaction mixture was stirred at 0°C for 30 minutes, acidified with 1N HCl and the resulting solid material was collected by filtration. The solid material was dissolved in methanol (2 ml) and THF (10 ml), and 1N NaOH (31 ml) was added. The mixture was stirred at room temperature for 3 hours and acidified with 1N HCl. The product was extracted into dichloromethane (100 ml × 3), washed with brine and dried over anhydrous sodium sulfate. The solvent was removed in vacuo and the residue in dichloromethane (30 ml) was treated with 5 drops of trifluoroacetic acid for 5 minutes. The solution was washed with saturated aqueous sodium bicarbonate, brine and dried over anhydrous sodium sulfate. Removal of the solvent gave the title compound as an oil.

NMR ($CDCl_3$) (ppm): 2.20—2.45 (3H, m), 2.80—2.95 (2H, m), 3.20—3.45 (2H, m), 3.60 (2H, t, J=6 Hz), 4.10 (2H, t, J=6 Hz), 5.82 (1H, d, J=6 Hz), 6.77—6.90 (2H, m), 7.35 (1H, d, J=8 Hz).

Example 22

5-(3-(4-acetyl-3-hydroxy-2-*n*-propylphenoxy)propyloxy)-1-hydroxyindane-2-acetic acid gamma lactone

A mixture of the compound of Example 21 (3.11 g), 2,4-dihyro-3-*n*-propylacetophenone (1.94 g) and potassium carbonate (2.76 g) in methyl ethyl ketone (40 ml) was refluxed for 6 hours. The reaction mixture was filtered and the filtrate was concentrated in vacuo. The residue was chromatographed on a column of silica gel (70—230 mesh, 100 g) eluting with hexane/EtOAc (1:1) to give the title compound as a solid, m.p. 96—97°.

| | | |
|---|---|---|
| Analysis, calculated: | C, 70.74; | H, 6.65. |
| Found: | C, 70.56; | H, 6.51. |

Example 23

5-(3-(4-Acetyl-3-hydroxy-2-*n*-propylphenoxy)propyloxy)-1-hydroxyindane-2-acetic acid sodium salt.

Following the procedure of Example 12, but substituting the compound of Example 22 for the compound of Example 11, the title compound was obtained as a solid, m.p. 156—159°.

| | | |
|---|---|---|
| Analysis, calculated: | C, 64.64; | H, 6.29. |
| Found: | C, 64.32; | H, 6.22. |

**Claims**

1. Compounds having the formula:

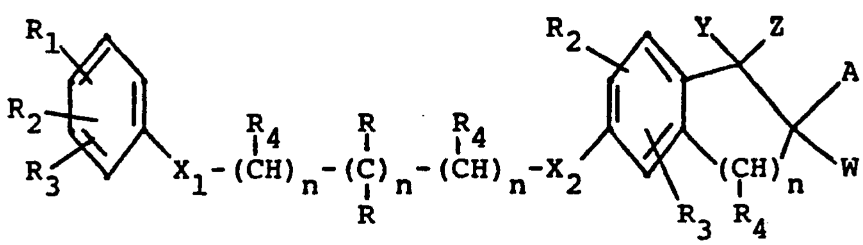

I

in which each R, independently of the other(s), is H, OH or $OR_2$, or $C(R)_2$ represents $C{=}C(R_4)_2$;

$R_1$ is H, OH, $R_4CO$, or $R_5OCO$;

each of $R_2$ and $R_3$, independently of the others, is H, OH, $C_{1-6}$ alkyl, $C_{2-6}$ alkenyl, trifluoromethyl, $C_{1-6}$ alkoxy, SH, $C_{1-6}$ thioalkyl, phenyl, phenyl having $C_{1-3}$ alkyl or halogen substitution, benzyl, phenethyl, halogen, amino, $N(R_4)_2$, $COOR_4$, $CH_2OR_4$, formyl, CN, trifluoromethylthio or nitro;

each of $X_1$ and $X_2$, independently of the other, is oxygen, sulfur, sulfoxide, sulfone, $—SO{=}NR_5$, $NR_6$, $N—CO—R_7$, N—CN or $NCONHR_6$;

each of $R_4$ and $R_6$, independently of the other(s), is H or $C_{1-6}$ alkyl;

each $R_5$ is $C_{1-6}$ alkyl;

each $R_7$, independently of the other, if any, is $C_{1-6}$ alkyl or $C_{1-6}$ alkoxy;

W is H or $C_{1-6}$ alkyl or is combined with Y to form an unsaturated bond;

Z is H, OH is $C_{1-6}$ alkoxy, or is combined with Y to form an oxo residue;

Y, if not combined with Z or W, is hydrogen;

A is $—(C(R_4)_2)_s—R_8$ where s is 0, 1, 2 or 3 and $R_8$ is $COOR_4$, $CH_2OH$, CHO, tetrazolyl, $NHSO_2R_9$, $CONHSO_2R_9$, hydroxymethylketone, CN, $CON(R_7)_2$, a monocyclic or bicyclic heterocyclic ring containing an acidic hydroxyl group, or

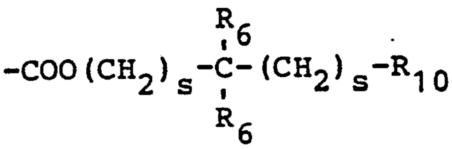

where each s, independently of the others, is 0, 1, 2 or 3 and $R_{10}$ is (A) a monocyclic or bicyclic heterocyclic radical containing from 3 to 12 nuclear carbon atoms and, as nuclear heteroatoms, two Ns or one N and one S, and with each ring in the heterocyclic radical being formed of 5 or 6 atoms, or (B) the radical $X'—R_{11}$ where X' is O, S or NH and $R_{11}$ contains up to 21 carbon atoms and is a hydrogen radical or an acyl radical of an organic acyclic or monocyclic carboxylic acid containing not more than 1 heteroatom in the ring;

each n, independently of the others, is 0, 1, 2 or 3

$R^9$ is OH, $C_{1-6}$ alkyl, $C_{1-6}$ alkoxy, $C_{1-6}$ perfluoroalkyl, phenyl, or phenyl having one or more $R^2$ substituents;

and compounds that are pharmaceutically acceptable lactones, salts or acid-addition salts theeof.

2. A compound as claimed in Claim 1 in which $X_1$ and $X_2$ are both oxygen.

3. A compound as claimed in Claim 1 in which one of $X_1$ and $X_2$ is oxygen and the other is sulfur, sulfoxide or sulfone.

4. A compound as claimed in Claim 1, 2 or 3, in which Z and Y are combined to form doubly bonded oxygen.

5. A compound as claimed in Claim 1, 2 or 3, in which Z is OH and Y is H.

6. A compound as claimed in Claim 1 in which in the formula,

$R_1$ is H, OH, or $R_4CO$;

..$X_1$ and $X_2$ are each independently oxygen, sulfur, sulfoxide or sulfone;

A is $—(C(R_4)_2)_s—R_8$ where s is 0, 1, 2 or 3 and $R_8$ is $COOR_4$, $CH_2OH$, CHO, tetrazolyl, $NHSO_2R_9$, $CONHSO_2R_9$, hydroxymethylketone, CN, or $CON(R)_7)_2$;

and the other variables are as defined in Claim 1.

7. A compound according to Claim 1 having the formula Ib:

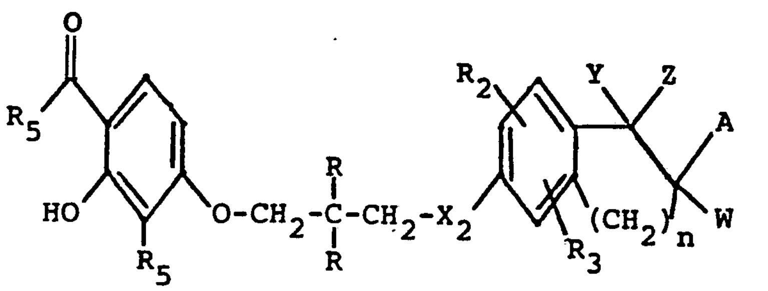

Ib

in which $X_2$ is oxygen, sulfur, sulfoxide or sulfone;

A is $—(C(R_4)_2)_s—R_8$ where s is 1, 2 or 3 and $R_8$ is $COOR_4$, $CH_2OH$, CHO, or tetrazolyl;

n is 1, 2 or 3,

and the other variables are as defined in Claim 1.

8. Methyl 5-[3-(4-acetyl-3-hydroxy-2-*n*-propylphenoxy)-propyloxy]-1-indanone-2-acetate.

9. 5-[3-(4-Acetyl-3-hydroxy-3-*n*-propylphenoxy)-propyloxy]-1-indanone-2-acetic acid.

10. Methyl 5-[3-(4-acetyl-3-hydroxy-2-*n*-propylphenoxy)-propylsulfonyl]-1-indanone-2-acetate.

11. 5-[3-(4-Acetyl-3-hydroxy-2-*n*-propylphenoxy)-propylsulfonyl]-1-indanone-2-acetic acid.

12. Methyl 5-[3-(4-acetyl-3-hydroxy-2-*n*-propylphenoxy)-propylthio]-1-indanone-2-acetate.

13. 5-[3-(4-Acetyl-3-hydroxy-2-*n*-propylphenoxy)-propylthio]-1-indanone-2-acetic acid.

14. 5-[3-(4-Acetyl-3-hydroxy-2-*n*-propylphenoxy)-propythio]-1-hydroxy-indane-2-acetic acid sodium salt.

15. 5-[3-(4-Acetyl-3-hydroxy-2-*n*-propylphenoxy)-propylthio]-1-hydroxy-indane-2-acetic acid γ-lactone.

16. Methyl 6-[3-(4-acetyl-3-hydroxy-2-*n*-propylphenoxy)-propylthio]-1-tetralone-2-acetate.

17. 6-[3-(4-Acetyl-3-hydroxy-2-*n*-propylphenoxy)-propylthiol-1-tetralone-2-acetic acid.

18. Methyl 6-[3-(4-acetyl-3-hydroxy-2-*n*-propylphenoxy)-propyloxy]-1-tetralone-2-acetate.

19. 6-[3-(4-Acetyl-3-hydroxy-2-*n*-propylphenoxy)-propyloxy]-1-tetralone-2-acetic acid.

20. 5-[3-(4-acetyl-3-hydroxy-2-*n*-propylphenoxy)-propyloxy]-1-hydroxyindane-2-acetic acid γ-lactone.

21. 5-[3-(4-Acetyl-3-hydroxy-2-*n*-propylphenoxy)-propyloxy]-1-hydroxyindane-2-acetic acid sodium salt.

22. A pharmaceutical composition, useful in antagonizing leukotriene action in mammals, comprising

a compound as claimed in any one of Claims 1 to 21 and a pharmaceutically acceptable carrier.

23. A composition as claimed in Claim 22, that also comprises a second active ingredient that is a non-steroidal anti-inflammatory drug; a peripheral analgesic agent; a cyclooxygenase inhibitor; a leukotriene antagonist; an antihistaminic agent; a prostaglandin antagonist or a thromboxane antagonist.

24. A composition as claimed in Claim 23 in which the second active ingredient is a non-steroidal anti-inflammatory drug.

25. A composition as claimed in Claim 24 in which the non-steroidal anti-inflammatory drug is indomethacin.

26. A composition as claimed in any one of Claims 23 to 25, in which the weight ratio of the compound of Claim 1 to the second active ingredient ranges from 200:1 to 1:200.

27. A compound as claimed in Claim 1 for use in antagonizing leukotriene action in a mammal, especially a human.

28. A compound as claimed in Claim 1 for use in inducing cytoprotection in a mammal.

29. A method of preparing a compound of formula I:

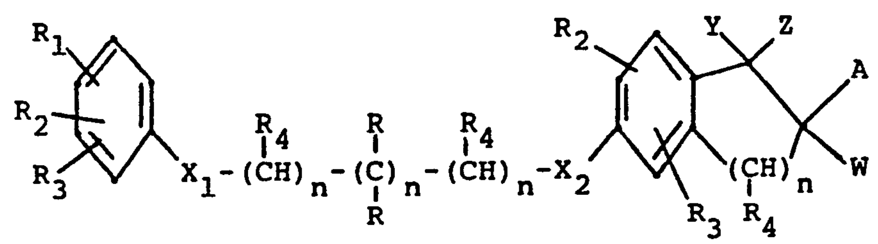

I

in which Z is OH and the other variables are as defined in Claim 1, that comprises reacting a compound having the formula XI':

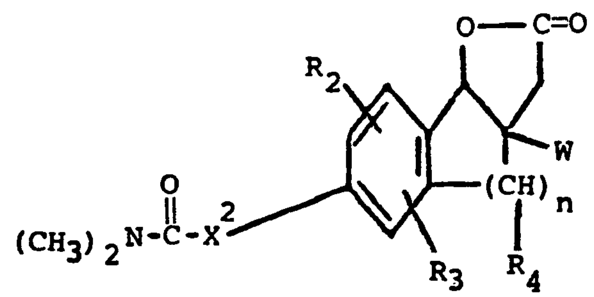

XI'

with a compound of formula VI':

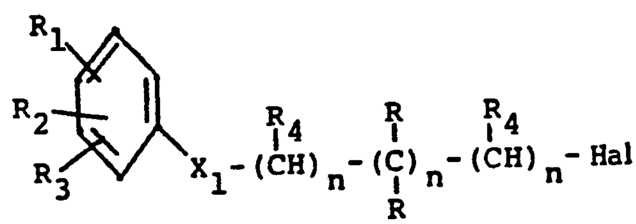

VI'

where Hal is a bromine, chlorine or iodine and the other variables are as defined in Claim 1.

30. A process for preparing compounds of the formula I:

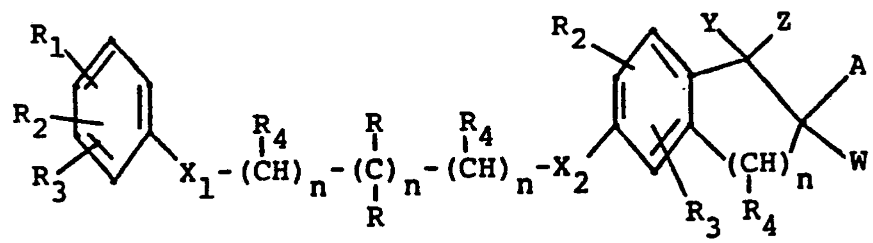

I

in which one of $X_1$ and $X_2$ is sulfur and the other substituents are as defined in Claim 1, that comprises reacting a compound having the formula VIII′:

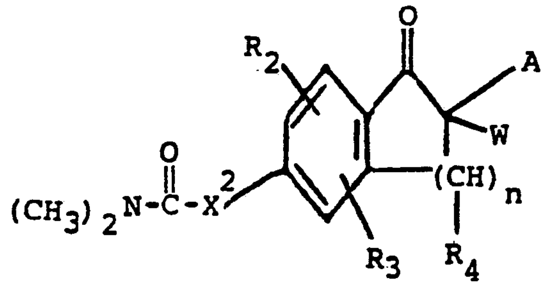

VIII′

with a compound of formula VI′:

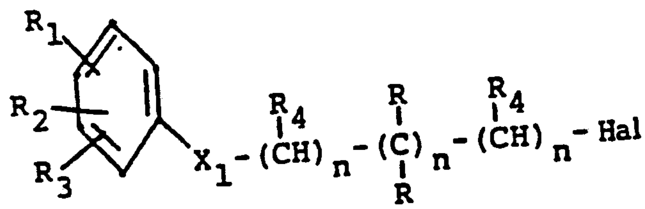

VI′

where $R_2$ is as defined in Claim 1.

**Patentansprüche**

1. Verbindungen mit der Formel

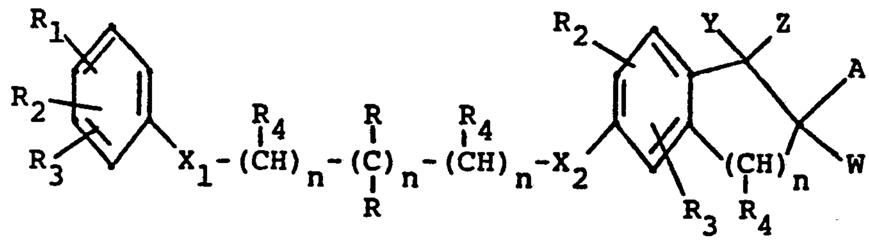

I

worin jedes R unabhängig von dem/den anderen H, OH oder $OR_2$ ist oder $C(R)_2\ C{=}C(R_4)_2$ darstellt;

$R_1$, H, OH, $R_4CO$ oder $R_5OCO$ ist;

jedes von $R_2$ und $R_3$ unabhängig von den anderen H, OH, $C_{1-6}$-Alkyl, $C_{2-6}$-Alkenyl, Trifluormethyl, $C_{1-6}$-Alkoxy, SH, $C_{1-6}$-Thioalkyl, Phenyl, Phenyl mit $C_{1-3}$-Alkyl- oder Halogensubstitution, Benzyl, Phenethyl, Halogen, Amino, $N(R_4)_2$, $COOR_4$, $CH_2OR_4$, Formyl, CN, Trifluormethylthio oder Nitro ist;

jedes von $X_1$ und $X_2$ unabhängig von den anderen Sauerstoff, Schwefel, Sulfoxid, Sulfon, $-SO{=}NR_5$, $NR_6$, $N-CO-R_7$, $N-CN$ oder $NCONHR_6$ ist;

jedes von $R_4$ und $R_6$, unabhängig von dem/den anderen H oder $C_{1-6}$-Alkyl ist;

jedes $R_5$ $C_{1-6}$-Alkyl ist;

jedes $R_7$ unabhängig von dem anderen, falls vorhanden, $C_{1-6}$-Alkyl oder $C_{1-6}$-Alkoxy ist;

W H oder $C_{1-6}$-Alkyl ist oder mit Y unter Bildung einer ungesättigten Bindung zusammengefaßt ist;

Z H, OH oder $C_{1-6}$-Alkoxy ist oder mit Y unter Bildung eines Oxorestes zusammengefaßt ist;

Y, falls nicht mit Z oder W zusammengefaßt, Wasserstoff ist;

A $-(C(R_4)_2)_s-R_8$ ist, worin s 0, 1, 2 oder 3 ist und $R_8$ $COOR_4$, $CH_2OH$, CHO, Tetrazolyl, $NHSO_2R_9$, $CONHSO_2R_9$, Hydroxymethylketon, CN, $CON(R_7)_2$, ein monocyclischer oder bicyclischer heterocyclischer Ring mit einer sauren Hydroxylgruppe ist oder

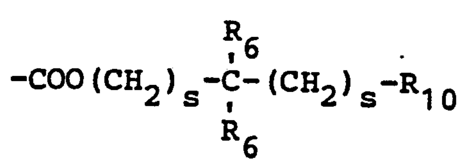

worin jedes s, unabhängig von den anderen, 0, 1, 2 oder 3 ist und $R_{10}$ (A) ein monocyclischer oder bicyclischer heterocyclischer Rest mit 3 bis 12 nuklearen Kohlenstoffatomen und als nuklearen Heteroatomen zwei N oder einem N und einem S ist, wobei jeder Ring in dem heterocyclischen Rest aus 5 oder 6 Atomen gebildet ist, oder (B) der Rest $X'—R_{11}$ ist, worin X', O, S oder NH ist und $R_{11}$ bis zu 21 Kohlenstoffatome enthält und ein Kohlenwasserstoffrest oder ein Acylrest einer organischen acylischen oder monocyclischen Carbonsäure mit nicht mehr als einem Heteroatom im Ring ist;

jedes n, unabhängig von den anderen, 0, 1, 2 oder 3 ist;

$R^9$ OH, $C_{1-6}$-Alkyl, $C_{1-6}$-Alkoxy, $C_{1-6}$-Perluoralkyl, Phenyl oder Phenyl mit einem oder mehreren $R^2$-Substituenten ist;

sowie Verbindungen, die pharmazeutisch annehmbare Lactone, Salze oder Säureadditionssalze davon sind.

2. Verbindung, wie in Anspruch 1 beansprucht, worin $X_1$ und $X_2$ beide Sauerstoff sind.

3. Verbindung, wie in Anspruch 1 beansprucht, worin eines von $X_1$ und $X_2$ Sauerstoff ist und das andere Schwefel, Sulfoxid oder Sulfon ist.

4. Verbindung, wie in Anspruch 1, 2 oder 3 beansprucht, worin Z und Y unter Bildung von doppelt gebundenem Sauerstoff zusammengenommen sind.

5. Verbindung, wie in Anspruch 1, 2 oder 3 beansprucht, worin Z OH ist und Y H ist.

6. Verbindung, wie in Anspruch 1 beansprucht, worin in der Formel

$R_1$ H, OH oder $R_4CO$ ist;

$X_1$ und $X_2$ jeweils unabhängig Sauerstoff, Schwefel, Sulfoxid oder Sulfon sind;

A $—(C(R_4)_2)_s—R_8$ ist, worin s 0, 1, 2 oder 3 ist und $R_8$ $COOR_4$, $CH_2OH$, CHO, Tetrazolyl, $NHSO_2R_9$, $CONHSO_2R_9$, Hydroxymethylketon, CN oder $CON(R_7)_2$ ist;

und die anderen Variablen wie in Anspruch 1 definiert sind.

7. Verbindung nach Anspruch 1 mit der Formel Ib:

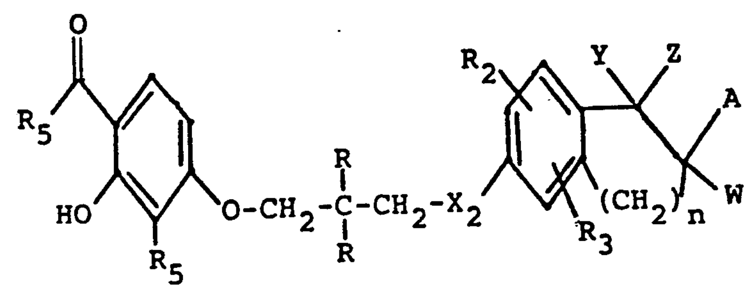

Ib

worin $X_2$ Sauerstoff, Schwefel, Sulfoxid oder Sulfon ist;

A $—(C(R_4)_2)_s—R_8$ ist, worin s 1, 2 oder 3 ist und $R_8$ $COOR_4$, $CH_2OH$, CHO oder Tetrazolyl ist;

n 1, 2 oder 3 ist;

und die anderen Variablen wie in Anspruch 1 definiert sind.

8. Methyl-5-[3-(4-acetyl-3-hydroxy-2-n-propylphenoxy)-propyloxy]-1-indanon-2-acetat.

9. 5-[3-(4-Acetyl-3-hydroxy-3-n-propylphenoxy)-propyloxy]-1-indanon-2-essigsäure.

10. Methyl-5-[3-(4-acetyl-3-hydroxy-2-n-propylphenoxy)-propylsulfonyl]-1-indanon-2-acetat.

11. 5-[3-(4-Acetyl-3-hydroxy-2-n-propylphenoxy)-propylsulfonyl]-1-indanon-2-essigsäure.

12. Methyl-5-[3-(4-acetyl-3-hydroxy-2-n-propylphenoxy)-propylthio]-1-indanon-2-acetat.

13. 5-[3-(4-Acetyl-3-hydroxy-2-n-propylphenoxy)-propylthio]-1-indanon-2-essigsaüre.

14. 5-[3-(4-Acetyl-3-hydroxy-2-n-propylphenoxy)-propylthio]-1-hydroxyindan-2-essigsäure, Natriumsalz.

15. 5-[3-(4-Acetyl-3-hydroxy-2-n-propylphenoxy)-propylthio]-1-hydroxyindan-2-essigsäure-γ-lacton.

16. Methyl-6-[3-(4-acetyl-3-hydroxy-2-n-propylphenoxy)-propylthio]-1-tetralon-2-acetat.

17. 6-[3-(4-Acetyl-3-hydroxy-2-n-propylphenoxy)-propylthio]-1-tetralon-2-essigsäure.

18. Methyl-6-[3-(4-acetyl-3-hydroxy-2-n-propylphenoxy)-propyloxy]-1-tetralon-2-acetat.

19. 6-[3-(4-Acetyl-3-hydroxy-2-n-propylphenoxy)-propyloxy]-1-tetralon-2-essigsäure.

20. 5-[3-(4-Acetyl-3-hydroxy-2-n-propylphenoxy)-propyloxy]-1-hydroxyindan-2-essigsäure-γ-lacton.

21. 5-[3-(4-Acetyl-3-hydroxy-2-n-propylphenoxy)-propyloxy]-1-hydroxyindan-2-essigsäure, Natriumsalz.

22. Pharmazeutische Zusammensetzung, geeignet zur Antagonisierung der Leukotrienwirkung in Säugetieren, welche eine Verbindung, wie in einem der Ansprüche 1 bis 21 beansprucht, sowie einen pharmazeutisch annehmbaren Träger umfaßt.

23. Zusammensetzung, wie in Anspruch 22 beansprucht, welche weiterhin einen zweiten wirksamen Bestandteil umfaßt, der ein nicht steroidales entzündungshemmendes Mittel; ein peripheres Analgetikum, ein Cyclooxygenaseinhibitor, ein Leukotrienantagonist; ein Antihistaminikum, ein Prostaglandinantagonist oder ein Thromboxanantagonist ist.

24. Zusammensetzung, wie in Anspruch 23 beansprucht, worin der zweite aktive Bestandteil ein nicht steroidales entzündungshemmendes Mittel ist.

25. Zusammensetzung, wie in Anspruch 24 beansprucht, worin das nicht steroidale entzündungshemmende Mittel Indomethacin ist.

26. Zusammensetzung, wie in einem der Ansprüche 23 bis 25 beansprucht, worin das Gewichtsverhältnis der Verbindung aus Anspruch 1 zum zweiten aktiven Bestandteil im Bereich von 200:1 bis 1:200 liegt.

27. Verbindung, wie Anspruch 1 beansprucht, zur Verwendung bei der Antagonisierung der Leukotrienwirkung in einem Säugetier, insbesondere im Menschen.

28. Verbindung, wie in Anspruch 1 beansprucht, zur Verwendung zur Induzierung eines Zellschutzes in einem Säugetier.

29. Verfahren zur Herstellung einer Verbindung der Formel I;

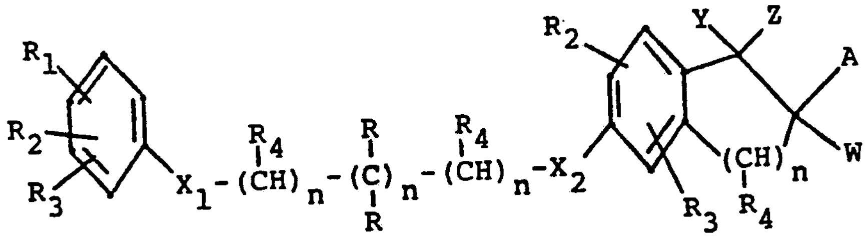

I

worin Z OH ist und die anderen Variablen wie in Anspruch 1 definiert sind, welches die Umsetzung einer Verbindung mit der Formel XI'

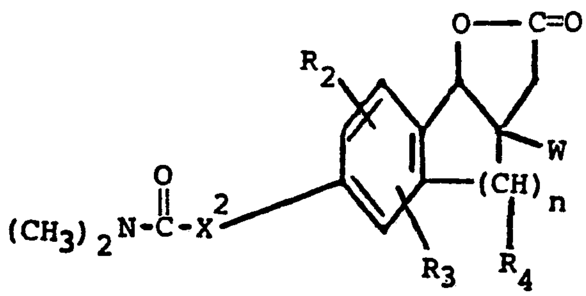

XI'

mit einer Verbindung der Formel VI'

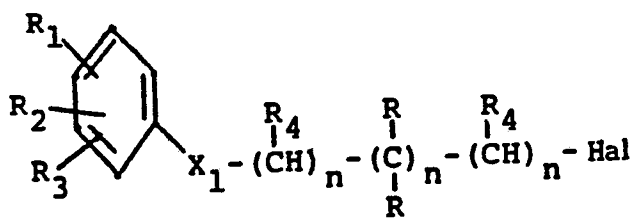

VI'

worin Hal Brom, Chlor oder Jod ist und die anderen Variablen wie in Anspruch 1 definiert sind, umfaßt.

30. Verfahren zur Herstellung von Verbindungen der Formel I

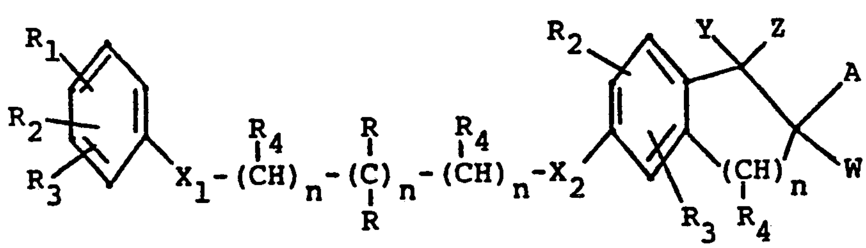

I

worin eines aus $X_1$ und $X_2$ Schwefel ist und die anderen Substituenten wie in Anspruch 1 definiert sind, welches die Umsetzung einer Verbindung mit der Formel VIII′

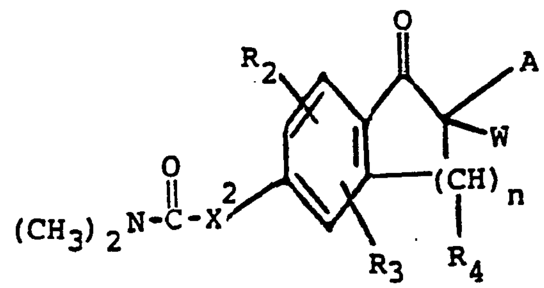

VIII′

mit einer Verbindung der Formel VI′

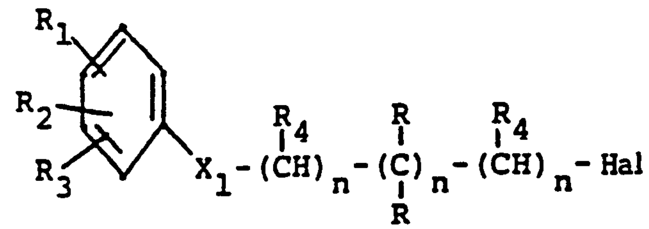

VI′

worin $R_2$ wie in Anspruch 1 definiert ist, umfaßt.

**Revendications**

1. Composé répondant à la formule:

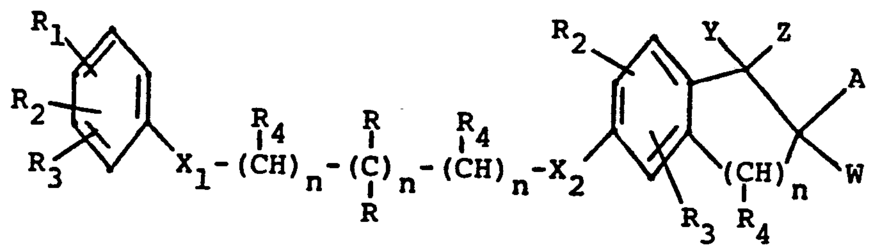

I

dans laquelle chaque R, indépendamment de l'autre ou des autres, est H, OH ou $OR_2$, ou $C(R)_2$ représente $C{=}C(R_4)2$;

$R_1$ est H, OH, $R_4CO$ ou $R_5OCO$;

chacun de $R_2$ et $R_3$, indépendamment des autres, est H, OH, un alkyle en $C_{1-6}$, un alcényle en $C_{2-6}$, un trifluorométhyle, un alcoxy en $C_{1-6}$, SH, un thioalkyle en $C_{1-6}$, un phényle, un phényle ayant une substitution alkyle en $C_{1-3}$ ou halogène, un benzyle, un phénéthyle, un halogène, un amino, $N(R_4)2$, $COOR_4$, $CH_2OR_4$, un formyle, CN, un trifluorométhylthio ou un nitro

chacun de $X_1$ et $X_2$, indépendamment de l'autre, est un oxygène, un soufre, un sulfoxyde, un sulfone, $—SO{=}NR_5$, $NR_6$, $N—CO—R_7$, N—CN ou $NCONHR_6$;

chacun de $R_4$ et $R_6$, indépendamment de l'autre ou des autres, est H ou un alkyle en $C_{1-6}$;

chaque $R_5$ et un alkyle en $C_{1-6}$;

chaque $R_7$, indépendamment de l'autre (éventuellement), est un alkyle en $C_{1-6}$, ou un alcoxy en $C_{1-6}$;

W est H ou un alkyle en $C_{1-6}$ ou est combiné avec Y pour former une liaison insaturée;

Z est H, OH ou un alcoxy en $C_{1-6}$, ou est combiné avec Y pour former un reste oxo;

Y, lorsqu'il n'est pas combiné avec Z ou W, est un hydrogène;

A est $—(C(R_4)_2)_s—R_8$, où s vaut 0, 1, 2 ou 3 et $R_8$ est $COOR_4$, $CH_2OH$, CHO, un tétrazolyle, $NHSO_2R_9$,

$CONHSO_2R_9$, une hydroxyméthylcétone, CN, $CON(R_7)_2$, un hétérocycle monocyclique ou bicyclique contenant un groupe hydroxyle acide ou

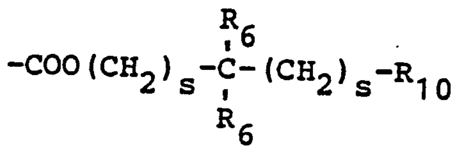

où chaque s, indépendamment des autres, vaut 0, 1, 2 ou 3 et $R_{10}$ est (A) un radical hétérocyclique monocyclique ou bicyclique contenant 3 à 12 atomes de carbone nucléaires et, comme hétéro-atomes nucléaires, deux N ou un N et n S, et chaque cycle du radical hétérocyclique est formé de 5 ou 6 atomes, ou (B) le radical $X'—R_{11}$ dans lequel X' est O, S ou NH et $R_{11}$ contient jusqu'à 21 atomes de carbone et est un radical hydrocarbure ou un radical acyle d'un acide organique carboxylique acyclique ou monocyclique ne contenant pas plus d'un hétéro-atome dans le cycle;

chaque n, indépendamment des autres, vaut 0, 1, 2 ou 3;

$R^9$ est OH, un alkyle en $C_{1-6}$, un alcoxy en $C_{1-6}$, un perfluoroalkyle en $C_{1-6}$, un phényle ou un phényle ayant un ou plusieurs substituants $R^2$;

et les composés qui sont leurs lactones, leurs sels ou leurs sels d'addition d'acides convenant en pharmacie.

2. Composé selon la revendication 1, où $X_1$ et $X_2$ sont tous deux un oxygène.

3. Composé selon la revendication 1, où un de $X_1$ et $X_2$ est un oxygène et l'autre est un soufre, un sulfoxyde ou une sulfone.

4. Composé selon la revendication 1, 2 ou 3, où Z et Y sont combinés pour former un oxygène à double liaison.

5. Composé selon la revendication 1, 2 ou 3, où Z est OH et Y est H.

6. Composé selon la revendication 1 où, dans la formule,

$R_1$ est H, OH ou $R_4CO$;

$X_1$ et $X_2$ sont chacun indépendamment un oxygène, un soufre, un sulfoxyde ou une sulfone.

A est $—(C(R_4)_2)_s—R_8$, où s vaut 0, 1, 2 ou 3 et $R_8$ est $COOR_4$, $CH_2OH$, CHO, un tétrazolyle, $NHSO_2R_9$, $CONHSO_2R_9$, une hydroxyméthylcétone, CN ou $CON(R_7)_2$;

et les autres variables sont comme défini dans la revendication 1.

7. Composé selon la revendication 1 répondant à la formule Ib:

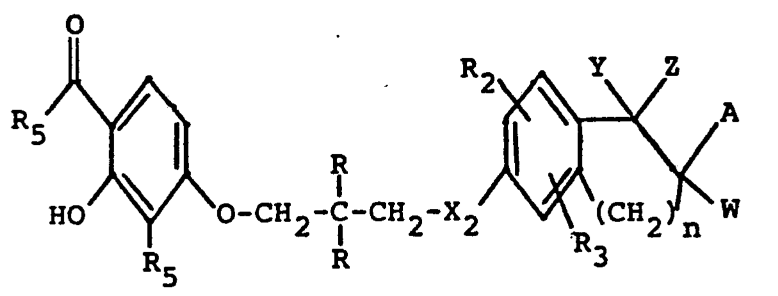

Ib

dans laquelle $X_2$ est un oxygène, un soufre, un sulfoxyde ou une sulfone;

A est $—(C(R_4)_2)_s—R_8$ où s vaut 1, 2 ou 3 et $R_8$ est $COOR_4$, $CH_2OH$, CHO ou un tétrazolyle;

n vaut 1, 2 ou 3,

et les autres variables sont comme défini dans la revendication 1.

8. 5-[3-(4-acétyl-3-hydroxy-2-n-propylphénoxy)propyloxy]-1-indanone-2-acétate de méthyle.

9. Acide 5-[3-(4-acétyl-3-hydroxy-3-n-propylphénoxy)propyloxy]-1-indanone-2-acétique.

10. 5-[3-(4-acétyl-3-hydroxy-2-n-propylphénoxy)propylsulfonyl]-1-indanone-2-acétate de méthyle.

11. Acide 5-[3-(4-acétyl-3-hydroxy-2-n-propylphénoxy)propylsulfonyl]-1-indanone-2-acétique.

12. 5-[3-(4-acétyl-3-hydroxy-2-n-propylphénoxy)propylthio]-1-indanone-2-acétate de méthyle.

13. Acide 5-[3-(4-acétyl-3-hydroxy-2-n-propylphénoxy)propylthio]-1-indanone-2-acétique.

14. Sel de sodium de l'acide 5-[3-(4-acétyl-3-hydroxy-2-n-propylphénoxy)propylthio]-1-hydroxyindane-2-acétique.

15. γ-lactone de l'acide 5-[3-(4-acétyl-3-hydroxy-2-n-propylphénoxy)propylthio]-1-hydroxyindane-2-acétique.

16. 6-[3-(4-acétyl-3-hydroxy-2-n-propylphénoxy)propylthio]-1-tétralone-2-acétate de méthyle.

17. Acide 6-[3-(4-acétyl-3-hydroxy-2-n-propylphénoxy)propylthio]-1-tétralone-2-acétique.

18. 6-[3-(4-acétyl-3-hydroxy-2-n-propylphénoxy)propyloxy]-1-tétralone-2-acétate de méthyle.

19. Acide 6-[3-(4-acétyl-3-hydroxy-2-n-propylphénoxy)propyloxy]-1-tétralone-2-acétique.

20. γ-lactone de l'acide 5-[3-(4-acétyl-3-hydroxy-2-n-propylphénoxy)propyloxy]-1-hydroxyindane-2-acétique.

21. Sel de sodium de l'acide 5-[3-(4-acétyl-3-hydroxy-2-n-propylphénoxy)propyloxy]-1-hydroxyindane-2-acétique.

22. Composition pharmaceutique utile pour contrecarrer l'action des leuctotriènes chez les mammifères, comprenant un composé selon l'une quelconque des revendications 1 à 21 et un véhicule convenant en pharmacie.

23. Composition selon la revendication 22, qui comprend aussi un second ingrédient actif qui est un médicament anti-inflammatoire non stéroïdien; un agent analgésique péripherique; un inhibiteur de la cyclooxygénase; un antagoniste des leucotriènes; un agent antihistaminique; un antagoniste de prostaglandine ou un antagoniste de thromboxane.

24. Composition selon la revendication 23, dans laquelle le second ingrédient est un médicament anti-inflammatoire non stéroïdien.

25. Composition selon la revendication 24, dans laquelle le médicament anti-inflammatoire non stéroïdien est l'indométhacine.

26. Composition selon l'une quelconque des revendications 23 à 25, dans laquelle le rapport pondéral du composé de la revendication 1 au second ingrédient actif se situe dans la gamme de 200/1 à 1/200.

27. Composition selon la revendication 1 pour l'emploi pour contrecarrer l'action d'un leucotriène chez un mammifère, en particulier un être humain.

28. Composé selon la revendication 1 pour l'emploi dans l'induction d'une cytoprotection chez un mammifère.

29. Procédé de préparation d'un composé de formule I:

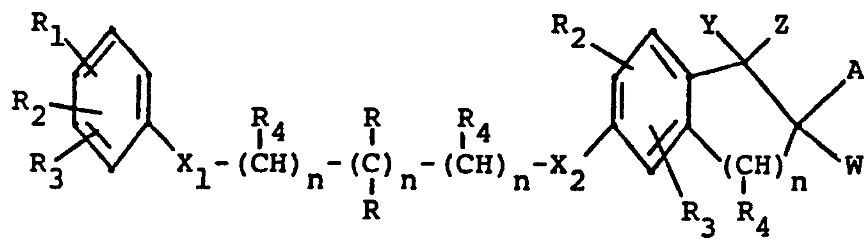

I

dans laquelle Z est OH et les autres variables sont comme défini dans la revendication 1, qui comprend la réaction d'un composé de formule XI':

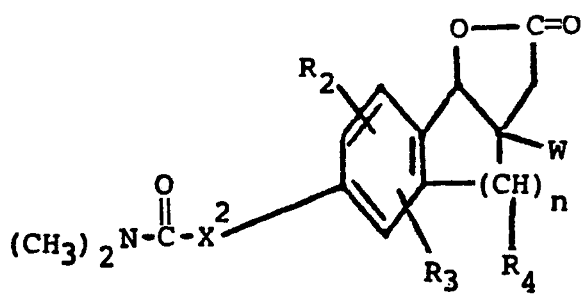

XI'

avec un composé de formule VI':

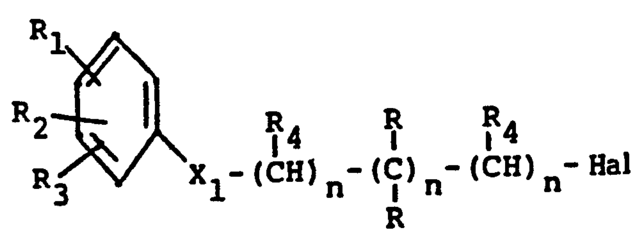

VI'

dans laquelle Hal est un brome, un chlore ou un iode et les autres variables sont comme défini dans la revendication 1.

30. Procédé pour préparer les composés de formule I:

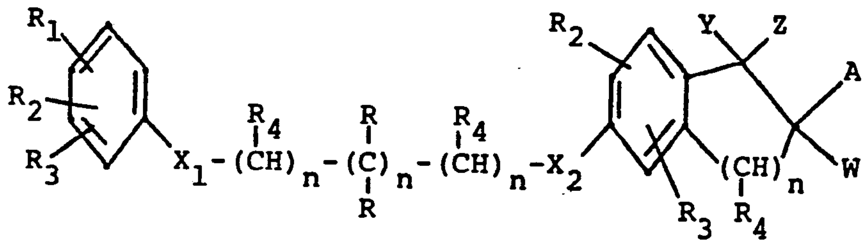

I

dans laquelle un de $X_1$ et $X_2$ est un soufre et les autres substituants sont comme défini dans la revendication 1, qui comprend la réaction d'un composé de formule VIII':

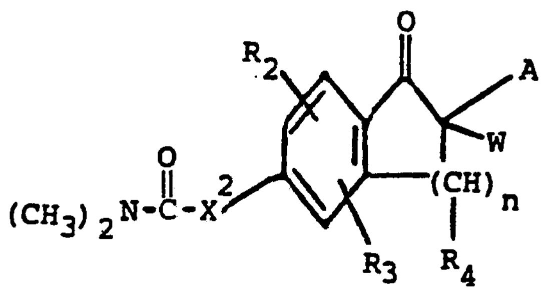

VIII'

avec un composé de formule VI':

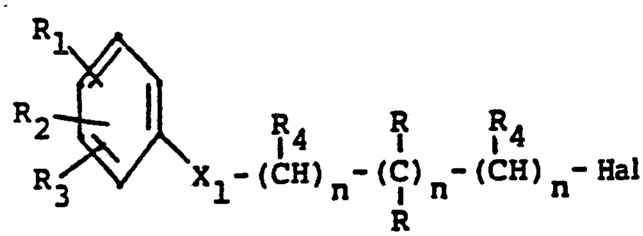

VI'

où $R_2$ est comme défini dans la revendication 1.